(19) Europäisches Patentamt
European Patent Office
Office européen des brevets

(11) EP 1 422 215 A1

(12) **EUROPEAN PATENT APPLICATION**
published in accordance with Art. 158(3) EPC

(43) Date of publication:
**26.05.2004 Bulletin 2004/22**

(21) Application number: **02755871.7**

(22) Date of filing: **08.08.2002**

(51) Int Cl.[7]: **C07C 51/363**, C07C 57/58, C07C 53/19, C07C 67/08, C07C 69/675, C07C 69/732, C07B 55/00, C07C 309/70

(86) International application number:
**PCT/JP2002/008098**

(87) International publication number:
**WO 2003/014056 (20.02.2003 Gazette 2003/08)**

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(30) Priority: **08.08.2001 JP 2001240672**
**10.07.2002 JP 2002201976**

(71) Applicant: **KANEKA CORPORATION**
**Osaka-shi, Osaka 530-8288 (JP)**

(72) Inventors:
- **YAMASHITA, Koki**
  **Osaka-shi, Osaka 550-0024 (JP)**
- **TAKEDA, Toshihiro**
  **Takasago-shi, Hyogo 676-0078 (JP)**
- **KINOSHITA, Koichi**
  **Kakogawa-shi, Hyogo 675-0057 (JP)**
- **UEDA, Yasuyoshi**
  **Himeji-shi, Hyogo 671-1227 (JP)**

(74) Representative: **VOSSIUS & PARTNER**
**Siebertstrasse 4**
**81675 München (DE)**

# (54) PROCESS FOR PRODUCING OPTICALLY ACTIVE 2-SUBSTITUTED CARBOXYLIC ACID

(57) The present invention relates to a process for efficiently producing an optically active 2-bromocarboxylic acid and an optically active 2-sulfonyloxycarboxylic acid, which are important in the production of medicinal compounds and so forth.

An optically active 2-sulfonyloxycarboxylic acid ester is subjected to deprotection under acid conditions to obtain an optically active 2-sulfonyloxycarboxylic acid. A metal bromide is caused to act on the acid to brominate it with configuration inversion at position 2 to thereby produce an optically active 2-bromocarboxylic acid. The resultant optically active 2-bromocarboxylic acid is isolated/purified by subjecting it to a step in which the acid is crystallized and separated as a salt with a base. Thus, an optically active 2-bromocarboxylic acid having a high chemical purity and high optical purity can be produced.

EP 1 422 215 A1

## Description

### TECHNICAL FIELD

The present invention relates to a process for producing an optically active 2-bromo carboxylic acid, an optically active 2-sulfonyloxycarboxylic acid, and an optically active 2-hydroxycarboxylic acid ester, in particular an (R)-2-bromocarboxylic acid, an (S)-2-sulfonyloxycarboxylic acid, and an (S)-2-hydroxycarboxylic acid ester.

The above-mentioned optically active 2-substituted carboxylic acid is useful as intermediates for the production of medicinal compounds and the like. In particular, an (R)-2-bromo-3-phenylpropionic acid, an (S)-2-sulfonyloxy-3-phenylpropionic acid, and an (S)-2-hydroxy-3-phenylpropionic acid ester are compounds useful as precursors of (S)-2-acetylthio-3-phenylpropionic acid, which is an intermediate for the production of an antihypertensive agent.

### BACKGROUND ART

A process for producing an optically active 2-bromocarboxylic acid which is known in the art comprises brominating an optically active amino acid using sodium nitrite while maintaining the configuration thereof (e.g. Japanese Kokai Publication Hei-08-337527). However, when this process is applied to a naturally occurring L-amino acid employed as starting materials, the product 2-bromocarboxylic acid has a (S) configuration at position 2. For producing a 2-bromocarboxylic acid having an (R) configuration at position 2, the process requires the use, as the starting materials, of a non-natural D-amino acid which are expensive and are not always available in large quantities. The above process also has a problem in that racemization may occur to a considerable extent with some amino acids.

Furthermore, an optically active 2-bromocarboxylic acid, typically an optically active 2-bromophenylpropionic acid, occur as oil in many instances and, therefore, it is not easy to isolate and purify them for increasing their chemical purity and optical purity, in particular optical purity.

Further, known in the art as the process for producing an optically active 2-sulfonyloxycarboxylic acid are:

i) a process comprising subjecting an optically active 2-sulfonyloxycarboxylic acid ester to alkaline hydrolysis (Japanese Kokai Publication Sho-58-10525), and

ii) a process comprising hydrolyzing a racemic 2-sulfonyloxycarboxylic acid ester using an enzyme (Japanese Kokai Publication Hei-10-14590), etc.

However, it was found that the above process i) has a problem in that certain optically active 2-sulfonyloxycarboxylic acid ester tends to be readily racemized. The above process ii) also has problems, for example, the yield and optical purity are not always satisfactory and the productivity is low, etc.

### SUMMARY OF THE INVENTION

In view of the above-discussed state of the art, it is an object of the present invention to produce an optically active 2-bromocarboxylic acid, an optically active 2-sulfonyloxycarboxylic acid, and an optically active 2-hydroxycarboxylic acid ester, which are important in the production of medicinal compounds and so forth, with high optical purity and high chemical purity in an economical and efficient manner.

As a result of intensive investigations made by them, the present inventors found that the use of an optically active 2-sulfonyloxycarboxylic acid as a reaction substrate and the bromination of the sulfonyloxy group thereof using a metal bromide with configuration inversion are favorable for the production of the corresponding optically active 2-bromocarboxylic acid high in optical purity and in chemical purity, that the coexisting optically active 2-sulfonyloxycarboxylic acid and/or optically active 2-hydroxycarboxylic acid can be efficiently removed by extracting and/or washing the optically active 2-bromocarboxylic acid produced with a mixed solvent system composed of an aromatic hydrocarbon and water and, further, that when the optically active 2-bromocarboxylic acid is converted to a salt with a base, it can be recovered as crystals and the coexisting impurities such as the optical isomer can be efficiently removed.

It was also found that the deprotection of an optically active 2-sulfonyloxycarboxylic acid ester under acidic conditions is particularly favorable for the preparation of the corresponding optically active 2-sulfonyloxycarboxylic acid, which is the reaction substrate, with high optical purity and high chemical purity.

Further, it was found that when the corresponding optically active 2-hydroxycarboxylic acid ester to serve as a reaction substrate is extracted and/or washed with a mixed solvent system composed of an organic solvent and water under weakly acidic to basic conditions in an after-treatment step following the esterification reaction, the coexisting optically active 2-hydroxycarboxylic acid can be efficiently removed and, in addition, that when crystallized from a solvent comprising an aliphatic hydrocarbon solvent, the optically active 2-hydroxycarboxylic acid ester can be recovered in the form of crystals and the optical isomer and like coexisting impurities can be thus removed.

Based on the above-mentioned series of findings, the present invention has been completed.

Thus, the present invention relates to

a process for producing an optically active 2-bromocarboxylic acid represented by the general formula (2) :

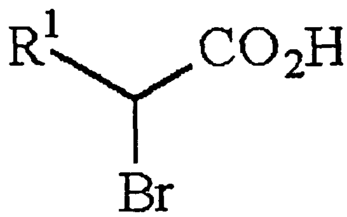

(2)

in the formula, $R^1$ represents an alkyl group containing 1 to 12 carbon atoms, which may optionally be substituted, an aryl group containing 6 to 14 carbon atoms, which may optionally be substituted, or an aralkyl group containing 7 to 15 carbon atoms, which may optionally be substituted,

which process comprises reacting an optically active 2-sulfonyloxycarboxylic acid represented by the general formula (1):

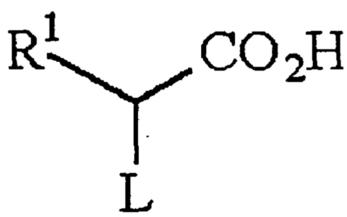

(1)

in the formula, $R^1$ is as defined above and L represents a sulfonyloxy group, with a metal bromide for bromination with configuration inversion at position 2.

The invention also relates to

a process for producing an optically active 2-bromocarboxylic acid,

which comprises extracting and/or washing an optically active 2-bromocarboxylic acid represented by the general formula (2) with a mixed solvent system composed of an aromatic hydrocarbon and water to thereby remove the corresponding coexisting optically active 2-sulfonyloxycarboxylic acid (1) and/or optically active 2-hydroxycarboxylic acid represented by the general formula (4):

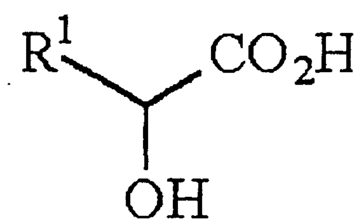

(4)

in the formula, $R^1$ is as defined above.

The invention further relates to

a process for isolating/purifying an optically active 2-bromocarboxylic acid,

which comprises, in isolating and purifying an optically active 2-bromocarboxylic acid represented by the general formula (2) and containing at least the optical isomer thereof as an impurity, the step of crystallizing and separating said optically active 2-bromocarboxylic acid in the form of a salt with a base. The invention is also concerned with crystals of a salt of the optically active 2-bromo-3-phenylpropionic acid with a base.

Further, the invention relates to

a process for producing an optically active 2-sulfonyloxycarboxylic acid represented by the general formula (1) which comprises deprotecting an optically active 2-sulfonyloxycarboxylic acid ester represented by the general

formula (3):

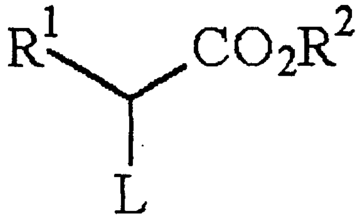

(3)

in the formula, $R^1$ and L are as defined above and $R^2$ represents a univalent organic group included in the structure represented by $-COOR^2$ and capable of serving as an ester type protective group for the carboxyl group, under acidic conditions.

In addition, the invention relates to

a process for producing an optically active 2-hydroxycarboxylic acid ester,

which comprises extracting and/or washing an optically active 2-hydroxycarboxylic acid ester represented by the general formula (5):

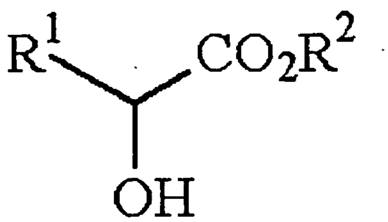

(5)

in the formula, $R^1$ and $R^2$ are as defined above, with a mixed solvent system composed of an organic solvent and water under weakly acidic to basic conditions to thereby eliminate the coexisting optically active 2-hydroxycarboxylic acid (4).

Furthermore, the invention relates to

a process for producing an optically active 2-hydroxycarboxylic acid ester,

which comprises crystallizing an optically active 2-hydroxycarboxylic acid ester represented by the general formula (5) and containing at least the optical isomer thereof as an impurity, using an aliphatic hydrocarbon solvent to recover said ester as crystals improved in optical purity. This process makes it possible to obtain a crystal of an optically pure and optically active 2-hydroxycarboxylic acid ester (5) in high yields and with good operability.

## DETAILED DISCLOSURE OF THE INVENTION

In the following, the present invention is described in detail.

In accordance with the invention, an optically active 2-bromocarboxylic acid represented by the general formula (2) is produced by reacting an optically active 2-sulfonyloxycarboxylic acid represented by the general formula (1) with a metal bromide compound for bromination with configuration inversion at position 2. The reaction substrate optically active 2-sulfonyloxycarboxylic acid (1) can be prepared, for example, by esterifying the corresponding optically active 2-hydroxycarboxylic acid represented by the general formula (4) to give an optically active 2-hydroxycarboxylic acid ester represented by the general formula (5), then treating this optically active 2-hydroxycarboxylic acid ester (5) with a leaving group introducing agent to give an optically active 2-sulfonyloxycarboxylic acid ester represented by the general formula (3) and, further, deprotecting this optically active 2-sulfonyloxycarboxylic acid ester (3) to give the optically active 2-sulfonyloxycarboxylic acid (1).

In the above general formulas (1), (2), (3), (4) and (5), $R^1$ represents an alkyl group containing 1 to 12 carbon atoms, which may optionally be substituted, an aryl group containing 6 to 14 carbon atoms, which may optionally be substituted, or an aralkyl group containing 7 to 15 carbon atoms, which may optionally be substituted.

The alkyl group containing 1 to 12 carbon atoms includes, for example, methyl, ethyl, isopropyl, tert-butyl, pentyl, hexyl, heptyl, n-octyl, nonyl, decyl group, etc.

The aryl group containing 6 to 14 carbon atoms includes, for example, phenyl, naphthyl group, and the like.

The aralkyl group containing 7 to 15 carbon atoms includes, for example, benzyl, phenylethyl, phenylpropyl group, and the like.

Referring to $R^1$, the substituent which each of the groups mentioned above may have includes, for example, alkoxy groups containing 1 to 12 carbon atoms, such as methoxy, ethoxy, tert-butyloxy, and n-octyloxy group; aryloxy groups containing 6 to 14 carbon atoms, such as phenyloxy and p-hydroxyphenyloxy group; aralkyloxy groups containing 7 to 15 carbon atoms, such as benzyloxy, p-chlorobenzyloxy, and p-fluorobenzyloxy group; acyl groups containing 1 to 15 carbon atoms, such as acetyl and benzoyl group; halogen atoms such as fluorine, chlorine, and bromine; hydroxyl group; amino group; carbamoyl group; guanidino group; imidazolyl group; indolyl group; cyclohexyl group; alkylthio groups containing 1 to 6 carbon atoms, such as methylthio and ethylthio group; phenylthio group; etc.

As specific examples of $R^1$, there may be mentioned methyl, ethyl, isopropyl, tert-butyl, n-octyl, hydroxymethyl, phenyl, p-hydroxyphenyl, benzyl, p-chlorobenzyl, p-fluorobenzyl, naphthyl group, and the like. An aralkyl group containing 7 to 15 carbon atoms, which may optionally be substituted, is preferred, and benzyl group is more preferred.

An organic group constituting the side chain of a natural amino acid or an organic group derived therefrom may preferably be used as the above group $R^1$. As the natural amino acid side chain-constituting organic group, there may be mentioned, for example, methyl, carbamoylmethyl, ethyl, carbamoylethyl, methylthioethyl, propyl, isopropyl, guanidinopropyl, isobutyl, sec-butyl, 4-aminobutyl, 5-aminopentyl, benzyl, p-hydroxybenzyl, 5-imidazolylmethyl, and 3-indolylmethyl group. As the organic group derived from such an amino acid side chain, there may be mentioned phenylthiomethyl, benzyloxymethyl, cyclohexylmethyl group, etc.

In the above general formulas (1) and (3), L represents a sulfonyloxy group. The sulfonyloxy group is not particularly restricted but includes, for example, a lower alkylsulfonyloxy group containing 1 to 4 carbon atoms, which may optionally be substituted, an arylsulfonyloxy group containing 6 to 10 carbon atoms, which may optionally be substituted, or a halosulfonyloxy group, and the like.

The lower alkylsulfonyloxy group containing 1 to 4 carbon atoms, which may optionally be substituted, is not particularly restricted but includes, for example, methanesulfonyloxy, ethanesulfonyloxy, trifluoromethanesulfonyloxy group, etc.

The arylsulfonyloxy group containing 6 to 10 carbon atoms, which may optionally be substituted, is not particularly restricted but includes, for example, benzenesulfonyloxy, p-chlorobenzenesulfonyloxy, p-toluenesulfonyloxy, o-, m-, or p-nitrobenzenesulfonyloxy group, etc.

The halosulfonyloxy group is, for example, chlorosulfonyloxy group, etc.

Among them, a lower alkylsulfonyloxy group containing 1 to 4 carbon atoms, and an arylsulfonyloxy group containing 6 to 10 carbon atoms, which may optionally be substituted, are preferred, and methanesulfonyloxy and p-chlorobenzenesulfonyloxy group are more preferred.

Even those sulfonyloxy groups which are not very high in leaving-group ability, for example methanesulfonyloxy, ethanesulfonyloxy, benzenesulfonyloxy, p-toluenesulfonyloxy group, etc., can be favorably used in the practice of the invention.

In the above general formulas (3) and (5), $R^2$ represents a univalent organic group included in the structure represented by $-COOR^2$ and capable of serving as an ester type, carboxyl-protective group. The term "protective group" as used herein means that the functional group in question is in a form modified so that no undesirable side reaction may occur.

The above univalent organic group is not particularly restricted but may be any of those effective in protecting the carboxyl group. For example, it can be selected from among those protective groups described in "Protective Groups in Organic Synthesis", 2nd Ed., published 1991 by John Wiley & Sons, Ltd. A lower alkyl group containing 1 to 4 carbon atoms (e.g. methyl, ethyl, n-propyl, isopropyl, n-butyl, sec-butyl, tert-butyl group) , benzyl group, a substituted benzyl group and the like are preferred among others, a lower alkyl group containing 1 to 4 carbon atoms is more preferred, and methyl group is still more preferred.

In the following, the respective steps according to the invention are described in detail.

First, the conversion of the optically active 2-hydroxycarboxylic acid (4) to the optically active 2-hydroxycarboxylic acid ester (5) can be accomplished by any of the esterification methods known in the art, for example by (a) the method comprising reacting the acid (4) with an alcohol in the presence of an inorganic acid such as sulfuric acid or hydrochloric acid, an organic acid such as sulfonic acid, e.g. methanesulfonic acid, p-toluenesulfonic acid, etc., or a Lewis acid such as boron fluoride etherate, etc., (b) the method comprising reacting the acid (4) with an O-alkylating agent such as diazomethane, (c) the method comprising reacting the acid (4) with an alkene or alkyne in the presence of an inorganic acid such as sulfuric acid or a Lewis acid such as boron trifluoride, (d) the method comprising reacting the acid (4) with an alkyl sulfate or alkyl halide in the presence of an inorganic base such as potassium carbonate or an organic base such as triethylamine or dimethylformamide, (e) the method comprising reacting the acid (4) with thionyl chloride and an alcohol, (f) the method comprising subjecting the acid (4) to transesterification with a fatty acid ester such as an acetic acid ester, or other methods.

For obtaining the optically active 2-hydroxycarboxylic acid ester (5) in a high quality form by esterification by

an industrially simple and easy technique while preventing contamination by the optically active 2-hydroxycarboxylic acid (4) after esterification, the process (a) or (e) mentioned above, namely the process comprising reacting the optically active 2-hydroxycarboxylic acid (4) with an alcohol and an acid or thionyl chloride, is preferred among others.

The alcohol species to be used in carrying out the above process (a) or (e) is not particularly restricted but preferably is an alcohol containing 1 to 10 carbon atoms, specifically methanol, ethanol, n-propanol, isopropanol, n-butanol, isobutanol, n-hexanol, cyclohexanol, benzyl alcohol, etc.

The amount of the above alcohol to be used is not particularly restricted but, generally, an amount of not less than 1 mole per mole of the optically active 2-hydroxycarboxylic acid (4) is preferred. Since this esterification is generally carried out by reacting the optically active 2-hydroxycarboxylic acid (4) with an alcohol and an acid or thionyl chloride in a large excess of an alcohol medium, the amount of the alcohol to be used is generally not less than about 5 moles per mole of the optically active 2-hydroxycarboxylic acid (4). The upper limit to the amount of the alcohol to be used is not particularly restricted but, from the economical viewpoint, it is generally not more than about 50 moles, preferably not more than about 30 moles, more preferably not more than about 20 moles, per mole of the acid.

For minimizing the amount of alcohol to be used and/or reducing the residual amount of the optically active 2-hydroxycarboxylic acid (4), the reaction may also be carried out while azeotropically removing the water produced by the reaction using an organic solvent capable of boiling in the form of an azeotrope with water, such as toluene.

In the case of method (a), the acid species to be used is not particularly restricted but includes, for example, inorganic acids such as hydrogen chloride and sulfuric acid; sulfonic acids such as methanesulfonic acid, ethanesulfonic acid, propanesulfonic acid, butanesulfonic acid, benzenesulfonic acid, p-toluenesulfonic acid, camphorsulfonic acid, and 1-phenylethanesulfonic acid; Lewis acids such as boron fluoride etherate, etc. Preferred are methanesulfonic acid and like sulfonic acids.

The amount of the above acid to be used is not particularly restricted but, generally, an amount of 0.01 to 100 moles, preferably 0.05 to 50 moles, more preferably 0.1 to 20 moles, per mole of the optically active 2-hydroxycarboxylic acid (4) .

In the case of method (e), the amount of thionyl chloride to be used is not particularly restricted but, generally, it is not less than 1 mole, preferably 1 to 5 moles, per mole of the optically active 2-hydroxycarboxylic acid (4).

In carrying out the above method (a) or (e), the three reagents, namely the above optically active 2-hydroxycarboxylic acid (4), the above alcohol, and the above acid or thionyl chloride, are generally brought into contact with one another at the same time. In the case of method (e) where the above thionyl chloride is used, it is also possible to react the above thionyl chloride with the above alcohol in advance, followed by reaction with the above optically active 2-hydroxycarboxylic acid (4).

The reaction solvent to be used in carrying out the method (a) or (e) is not particularly restricted. The above alcohol may be used also as the reaction solvent concurrently, aliphatic hydrocarbons such as hexane and heptane, aromatic hydrocarbons such as toluene, halogenated hydrocarbons such as methylene chloride, ethers such as tetrahydrofuran and tert-butyl methyl ether, etc., may also be used as the reaction solvent.

The reaction temperature in carrying out the method (a) or (e) is not particularly restricted but, generally, it is within the range of from the solidification temperature of the reaction mixture to about 100°C, preferably within the range of -20 to 80°C.

The reaction can be completed generally within 24 hours, preferably within 12 hours.

For minimizing the formation of the byproduct 2-hydroxycarboxylic acid (4) during after-treatment such as the extraction operation after reaction and reducing the load to be borne for impurity elimination in such an after-treatment procedure as the extraction operation, which is to be described later herein, the method (a) is more preferred.

Now, the process of obtaining an organic solvent layer containing the optically active 2-hydroxycarboxylic acid ester (5) is described.

In the esterification reaction mixture obtained in the above manner, there coexist, as impurities, the unreacted optically active 2-hydroxycarboxylic acid (4), an inorganic acid such as sulfuric acid or hydrochloric acid, and a sulfonic acid such as methanesulfonic acid in addition to the desired product optically active 2-hydroxycarboxylic acid ester (5).

The optically active 2-hydroxycarboxylic acid ester (5) may be obtained at a high quality level by neutralizing the reaction mixture or a concentrate derived therefrom with a base and extracting and/or washing the neutralization mixture using an organic solvent and water to thereby eliminate the unreacted optically active 2-hydroxycarboylic acid (4) in the aqueous layer. The above operation can prevent the desired product (5) from being contaminated with the optically active 2-hydroxycarboxylic acid (4) otherwise readily accompanying the product (5).

As for the above procedure, the esterification reaction mixture after neutralization may be extracted with an organic solvent, or the esterification reaction mixture may be admixed with an organic solvent, followed by neutralization and extraction, for instance. In the above procedure, it is also possible to carry out the extraction operation after partly or entirely removing the alcohol component in the esterification reaction mixture. From the viewpoint of stabilization of the pH value indicated in the step of neutralization (pH adjustment) , however, the neutralization is preferably carried out in the presence of the alcohol component.

The extraction solvent is not particularly restricted but preferably is a hydrocarbon solvent, an ester solvent, or an ether solvent, more preferably a hydrocarbon solvent or an ester solvent, in particular a hydrocarbon solvent.
The hydrocarbon solvent is preferably an aromatic hydrocarbon solvent, an aliphatic hydrocarbon solvent, or a halogenated hydrocarbon solvent, more preferably an aromatic hydrocarbon solvent or an aliphatic hydrocarbon solvent.
The aromatic hydrocarbon solvent is not particularly restricted but includes aromatic hydrocarbons preferably containing 6 to 12 carbon atoms, more preferably 6 to 10 carbon atoms, still more preferably 6 to 8 carbon atoms, specifically benzene, toluene, xylene, etc. Among them, aromatic hydrocarbons containing 7 or 8 carbon atoms, specifically toluene, xylene and the like, are preferred, and toluene is most preferably used.
The aliphatic hydrocarbon solvent is not particularly restricted but includes aliphatic hydrocarbons preferably containing 5 to 12 carbon atoms, more preferably 5 to 8 carbon atoms, specifically, for example, pentane, hexane, heptane, methylcyclohexane, etc. Among them, aliphatic hydrocarbons containing 6 or 7 carbon atoms, specifically hexane, heptane, methylcyclohexane and the like, are preferred, and hexane and heptane are most preferably used.
The halogenated hydrocarbon solvent is not particularly restricted but includes, as preferred species, methylene chloride, chlorobenzene, dichlorobenzene, 1,2-dichloroethane, and the like.
The ester solvent is not particularly restricted but includes, as preferred species, esters containing 2 to 8 carbon atoms, more preferably 3 to 5 carbon atoms, specifically, for example, ethyl acetate, n-propyl acetate, isopropyl acetate, n-butyl acetate, tert-butyl acetate, etc. Among them, ethyl acetate is judiciously used.
The ether solvent is not particularly restricted but preferably is an acyclic ether solvent, etc. Preferred as the acyclic ether solvent are methyl tert-butyl ether, dibutyl ether and the like. Methyl tert-butyl ether is preferred among others.
It is of course possible to use the above-mentioned solvents in the form of a mixed solvent composed of two or more species.
The above extraction and/or washing operation can be carried out under weakly acidic to basic conditions. The lower limit to pH is generally not lower than 4, preferably not lower than 5, more preferably not lower than 6, still more preferably not lower than 7, and the upper limit is generally not higher than 14, preferably not higher than 13, more preferably not higher than 12, still more preferably not higher than 11 although it depends on the temperature.
The base to be used for neutralizing the esterification reaction mixture or a condensate thereof may be either an inorganic base or an organic base but preferably is an inorganic base. Specifically, the inorganic base includes, but is not limited to, alkali metal hydroxides such as sodium hydroxide and potassium hydroxide; alkali metal carbonates such as sodium carbonate and potassium carbonate; alkali metal hydrogencarbonates such as sodium hydrogencarbonate; etc. Among them, alkali metal hydrogencarbonates are preferred, and sodium hydrogencarbonate is most preferred.
The above extraction may suitably include a washing operation, which is preferably carried out under the conditions mentioned above for the extraction. Generally, the washing operation can be preferably carried out under the weakly acidic to basic conditions mentioned above although the conditions may vary according to the temperature.
The optically active 2-hydroxycarboxylic acid ester (5) can be recovered from the extract or washings obtained in the above manner by distilling off the reaction solvent or extraction solvent by such an operation as heating under reduced pressure while preventing the optically active 2-hydroxycarboxylic acid (4) from mixing therein. The thus-obtained optically active 2-hydroxycarboxylic acid ester (5) is nearly pure but may further be increased in purity by an additional conventional technique such as purification by crystallization or column chromatography.
The solvent to be used in crystallizing the optically active 2-hydroxycarboxylic acid ester (5) is not particularly restricted but preferably is a hydrocarbon solvent such as an aliphatic hydrocarbon solvent, an aromatic hydrocarbon solvent or a halogenated hydrocarbon solvent, an ester solvent, or an ether solvent. Especially when the crystallization is carried out using a hydrocarbon solvent, in particular an aliphatic hydrocarbon solvent, it is possible to obtain the optically active 2-hydroxycarboxylic acid ester (5) in the form of crystals improved in purity from the optically active 2-hydroxycarboxylic acid ester (5) containing at least its optical isomer as an impurity.
The aliphatic hydrocarbon solvent is not particularly restricted but includes aliphatic hydrocarbons preferably containing 5 to 12 carbon atoms, more preferably 5 to 8 carbon atoms, specifically, for example, pentane, hexane, heptane, methylcyclohexane, etc. Among them, aliphatic hydrocarbons containing 6 or 7 carbon atoms, specifically hexane, heptane and methylcyclohexane, are preferred.
The aromatic hydrocarbon solvent is not particularly restricted but includes aromatic hydrocarbons preferably containing 6 to 12 carbon atoms, more preferably 6 to 10 carbon atoms, still more preferably 6 to 8 carbon atoms, specifically, for example, benzene, toluene, xylene, etc. Among them, aromatic hydrocarbons containing 7 or 8 carbon atoms, specifically toluene, xylene, etc., are preferred, and toluene is most preferably used.
The halogenated hydrocarbon solvent is not particularly restricted but preferably includes methylene chloride, chlorobenzene, dichlorobenzene, 1,2-dichloroethane and the like. Among them, methylene chloride is preferred.
The ester solvent is not particularly restricted but includes esters preferably containing 2 to 8 carbon atoms,

more preferably 3 to 5 carbon atoms, specifically, for example, ethyl acetate, n-propyl acetate, isopropyl acetate, n-butyl acetate, tert-butyl acetate and the like. Among them, ethyl acetate is preferred.

The ether solvent is not particularly restricted but preferably is an acyclic ether solvent or the like. The acyclic ether solvent includes, as preferred species, methyl tert-butyl ether, dibutyl ether and the like. Among them, methyl tert-butyl ether is preferred, however.

The aliphatic hydrocarbon solvents mentioned above may be used singly or in the form of a mixed solvent composed of two or more species. In particular, the combined use of the above-mentioned aromatic hydrocarbon solvent and/or ester solvent is an advantageous means of improving the productivity through an improvement in crystallization concentration and of achieving an improvement in purity of the optically active 2-hydroxycarboxylic acid ester (5).

Thus, in crystallizing the optically active 2-hydroxycarboxylic acid ester (5), the crystallization is carried out using the above-mentioned aliphatic hydrocarbon solvent as a major solvent as judged at the time of completion of crystallization, optionally combinedly using the aromatic hydrocarbon solvent and/or ester solvent as an auxiliary solvent.

The phrase "using as a major solvent" as used herein means that the solvent in question amounts to a major volume in the whole solvent volume and, generally, that solvent preferably amounts to not less than half the whole solvent volume. The amount of the above aliphatic hydrocarbon solvent at the time of completion of crystallization is more preferably not less than 2/3 by volume, still more preferably not less than 4/5 by volume, still further preferably not less than 9/10 by volume, most preferably not less than 95/100 by volume, and such amount is preferably reached by successive addition of the aliphatic hydrocarbon solvent, for instance.

On the other hand, the phrase "combinedly using as an auxiliary solvent" means that the solvent in question amounts to a volume smaller than that of the main solvent. Generally, that amount is less than half.the whole solvent volume.

In the practice of the invention, the crystallization operation can be carried out using any of the processes known in the art, such as cooling crystallization and concentration crystallization, or a combination of two or more known processes.

The crystallization temperature is not particularly restricted but, generally, it is not higher than 60°C, preferably not higher than 40°C, more preferably not higher than 20°C, and the lower limit is the solidification temperature of the system. Generally, the crystallization can be adequately carried out at -20 to 40°C, preferably about -10 to 20°C.

In carrying out the crystallization, seed crystals may be added, if necessary, for promoting nucleation.

The crystallization is preferably carried out under stirring. The power required for stirring per unit volume is not particularly restricted but, preferably, the crystallization is carried out with stirring by a power of not less than 0.05 $kW/m^3$, preferably not less than 0.1 $kW/m^3$, more preferably not less than 0.3 $kW/m^3$, for instance.

The thus-formed crystals of the optically active 2-hydroxycarboxylic acid ester (5) can be recovered by an ordinary solid-liquid separation technique, such as centrifugation, pressure filtration or vacuum filtration. Where necessary, the crystals obtained may be further subjected to drying under reduced pressure (vacuum drying) to give dry crystals.

The optically active 2-hydroxycarboxylic acid ester (5) is then treated with a leaving group introducing agent, whereby the corresponding optically active 2-sulfonyloxycarboxylic acid ester (3) can be obtained.

The leaving group introducing agent is not particularly restricted but includes, for example, methanesulfonyl chloride, ethanesulfonyl chloride, trifluoromethanesulfonyl chloride, benzenesulfonyl chloride, p-chlorobenzenesulfonyl chloride, p-toluenesulfonyl chloride, o-nitrobenzenesulfonyl chloride, m-nitrobenzenesulfonyl chloride, p-nitrobenzenesulfonyl chloride, etc. Methanesulfonyl chloride and p-chlorobenzenesulfonyl chloride are preferred, however.

The reaction solvent is not particularly restricted but includes, for example, aromatic hydrocarbon solvents such as toluene.

The reaction temperature is not particularly restricted but, generally, it is -20 to 80°C, preferably 0 to 50°C. The reaction time is not particularly restricted but, generally, it is 0.1 to 48 hours, preferably 1 to 24 hours.

And, the conversion of the optically active 2-sulfonyloxycarboxylic acid ester (3) to the corresponding optically active 2-sulfonyloxycarboxylic acid (1) may be carried out by any of the deprotection methods known for other ester compounds, for example by (a) the method comprising effecting hydrolysis under acidic conditions (acid hydrolysis) using an acid, for example an inorganic acid such as sulfuric acid, hydrochloric acid or hydrobromic acid, an sulfonic acid such as methanesulfonic acid or p-toluenesulfonic acid, or a carboxylic acid such as formic acid, acetic acid or trifluoroacetic acid, (b) the method comprising effecting hydrolysis under basic conditions (base hydrolysis) using a base, for example an alkali metal hydroxide such as sodium hydroxide or potassium hydroxide, an alkaline earth metal hydroxide such as barium hydroxide, an alkali metal carbonate such as sodium carbonate, an organic base such as triethylamine, imidazole or an amidine, or an alkali metal alkoxide such as sodium methoxide or potassium tert-butoxide, (c) the method comprising effecting hydrolysis of silyl esters, such as a trimethylsilyl ester, under neutral conditions (neutral hydrolysis) using water, an alcohol, etc., (d) the method comprising effecting catalytic reduction (reductive

degradation) of aralkyl esters, such as a benzyl esters, using a noble metal catalyst such as palladium, or other methods.

For preventing a decrease in optical purity of the optically active 2-sulfonyloxycarboxylic acid (1) as caused by racemization thereof, however, the above-mentioned methods (a), (c) and (d) are preferably used, the methods (a) and (d) are more preferred, and the acid hydrolysis method (a) is most preferred. When this method is employed, the optically active 2-sulfonyloxycarboxylic acid (1) can be obtained in a form high in optical purity.

In carrying out the above acid hydrolysis (a), the method comprising reacting the ester (3) with a carboxylic acid in the presence of a strong acid, or the method comprising carrying out the hydrolysis in an aqueous medium containing an organic solvent highly compatible with water in the presence of a strong acid.

The strong acid is not particularly restricted but may be, for example, an inorganic acid such as sulfuric acid, a sulfonic acid such as methanesulfonic acid, etc., preferably an inorganic acid such as sulfuric acid. The amount of the strong acid to be used may be a catalytic amount or larger. Generally, it is used in an amount of 0.01 to 100 moles, preferably 0.05 to 50 moles, more preferably 0.1 to 20 moles, per mole of the optically active 2-sulfonyloxycarboxylic acid ester (3).

When the ester is reacted with a carboxylic acid in the presence of a strong acid, the carboxylic acid may be, for example, formic acid, acetic acid, propionicacid, etc., formic acid being particularly preferred. The carboxylic acid is used in an amount of not less than 1 mole, preferably not less than 3 moles, per mole of the optically active 2-sulfonyloxycarboxylic acid ester (3).

In carrying out the reaction in an aqueous medium containing an organic solvent highly compatible with water in the presence of a strong acid, the highly water-compatible organic solvent to be used is not particularly restricted but includes, for example, dioxane, methanol, ethanol, etc.

In the above-mentioned acid hydrolysis, water is used generally in an amount of not less than 1 mole, preferably not less than 5 moles, still more preferably not less than 20 moles, per mole of the optically active 2-sulfonyloxycarboxylic acid ester (3).

The reaction solvent is not particularly restricted but may be any of such substantially inert solvents as aliphatic hydrocarbons such as heptane, aromatic hydrocarbons such as toluene, halogenated hydrocarbons such as methylene chloride, ethers such as tetrahydrofuran, dioxane and tert-butyl methyl ether, and alcohols such as methanol and ethanol, for instance. It is of course possible to use water in an excessive amount so that it may also serve as a reaction solvent.

The reaction is generally carried out with warming, judiciously at 40°C or above, for instance, preferably at 60°C or above. The reaction can be completed generally in 24 hours, preferably in 12 hours.

In the above deprotection, the rate of configuration retention is generally not less than 90%, preferably not less than 95%, more preferably not less than 97%, still more preferably not less than 99%. The rate of configuration retention so referred to herein is expressed in terms of the ratio of the enantiomer excess (% ee) of the product to the enantiomer excess (% ee) of the starting material having the same configuration.

After the reaction, the product can be recovered from the reaction mixture by after-treatment in the conventional manner. For example, the mixture after completion of the reaction, if necessary after concentration, is extracted with a conventional extraction solvent. The extraction solvent includes, for example, toluene, ethyl acetate, tert-butyl methyl ether, methylene chloride, etc. Using tert-butyl methyl ether, among them, makes it possible to extract the desired product (1) very efficiently.

For the purpose of removing impurities, for instance, the reaction mixture or a concentrate thereof may be neutralized with a base, whereby the salt of the optically active 2-sulfonyloxycarboxylic acid (1) may be recovered in the form of a salt with the base. The thus-obtained salt of the optically active 2-sulfonyloxycarboxylic acid (1) with the base can be converted to the free optically-active 2-sulfonyloxycarboxylic acid (1) by neutralization with an acid higher in acidity than the 2-sulfonyloxycarboxylic acid, for example an inorganic acid such as hydrochloric acid or sulfuric acid, etc. This free-form optically active 2-sulfonyloxycarboxylic acid (1) can be extracted with an organic solvent, if necessary followed by removal of the solvent, to give the optically active 2-sulfonyloxycarboxylic acid (1) or a solution thereof.

In that case, the base to be used for neutralization includes basic alkali metal compounds and alkaline earth metal compounds including alkali metal hydroxides such as lithium hydroxide, sodium hydroxide and potassium hydroxide, alkaline earth metal hydroxides such as barium hydroxide, alkali metal carbonates such as sodium carbonate and potassium carbonate, alkali metal hydrogencarbonates such as sodium hydrogencarbonate and potassium hydrogencarbonate, and the like, etc. Among them, alkali metal hydroxides are preferred, and sodium hydroxide is most preferred.

The above neutralization with a base is preferably carried out under weakly acidic to basic conditions in the presence of water. When the conditions are defined in terms of pH, the pH is generally not lower than 4, preferably not lower than 5, more preferably not lower than 6, most preferably not lower than 7.

The salt of the optically active 2-sulfonyloxycarboxylic acid (1) with the above base as formed in the above manner may be recovered in each form of an aqueous solution or crystals. After recovery, the salt is converted to the

optically active 2-sulfonyloxycarboxylic acid (1), and the product is extracted with the extraction solvent mentioned above.

The optically active 2-sulfonyloxycarboxylic acid (1) can be obtained by removing the reaction solvent and/or extraction solvent from the extract by such an operation as heating under reduced pressure. The thus-obtained optically active 2-sulfonyloxycarboxylic acid (1) is almost pure. It is also possible, however, to further purify the acid (1) by such a conventional technique as crystallization purification, distillation purification, or column chromatography to attain a higher purity.

In crystallizing the optically active 2-sulfonyloxycarboxylic acid (1), toluene is preferably used as the crystallization solvent. The purpose of purification by crystallization can be accomplished very efficiently by substituting the reaction solvent (preferably tert-butyl methyl ether) with the crystallization solvent toluene to thereby cause crystallization.

The process for producing the optically active 2-bromocarboxylic acid (2) by reacting the optically active 2-sulfonyloxycarboxylic acid (1) with a metal bromide for bromination with configuration inversion at position 2 is now described.

The above bromination with configuration inversion is carried out using a metal bromide. The metal bromide includes, for example, alkali metal bromides such as lithium bromide, sodium bromide and potassium bromide, alkaline earth metal bromides such as magnesium bromide and calcium bromide, aluminum bromide, etc. Alkali metal bromides are preferred, and lithium bromide is most preferred.

The amount of the above metal bromide to be used is not particularly restricted but, generally, it is not less than the theoretically equivalent amount. From the viewpoint of economy, reactivity, and/or the like, it is preferably 1 to 5 times, more preferably 1 to 3 times, the theoretically equivalent amount. When lithium bromide, which is the most preferred metal bromide, is used, its amount to be used is generally not less than 1 mole, preferably 1 to 5 moles, more preferably 1 to 3 moles, still more preferably 1 to 1.5 moles, per mole of the optically active 2-sulfonyloxycarboxylic acid (1). Unless the reaction yield is adversely affected, an amount as close to 1 mole as possible is preferred.

The reaction solvent is not particularly restricted but may be any of the essentially inert solvents. For preventing the optical purity from lowering as a result of racemization, however, a hydrocarbon or an ether is preferably used. More specifically, the hydrocarbon may be an aliphatic hydrocarbon (preferably an aliphatic hydrocarbon containing 5 to 12 carbon atoms, more preferably 5 to 8 carbon atoms), an aromatic hydrocarbon (preferably an aromatic hydrocarbon containing 6 to 12 carbon atoms, more preferably 6 to 10 carbon atoms), or a halogenated hydrocarbon (preferably a halogenated hydrocarbon (in particular a chlorinated hydrocarbon) containing 1 to 6 carbon atoms, more preferably 1 to 4 carbon atoms). As for the ether, an acyclic ether (preferably an ether containing 4 to 12 carbon atoms, more preferably 4 to 8 carbon atoms) is preferably used, among others.

The aliphatic hydrocarbon mentioned above includes, for example, hexane, heptane, methylcyclohexane, etc., the aromatic hydrocarbon includes, for example, benzene, toluene, etc., and the halogenated hydrocarbon includes, for example, methylene chloride, 1,2-dichloroethylene, 1,1,2-trichloroethylene, etc. The ether mentioned above includes, for example, tert-butyl methyl ether, diisopropyl ether and like ethers. Among the solvents mentioned above, aromatic hydrocarbons or halogenated hydrocarbons are particularly preferred.

The reaction temperature may vary depending on the metal bromide species employed, the amount thereof, and the reaction solvent species, hence cannot be specified absolutely. Generally, however, it is not lower than room temperature, for example not lower than about 10°C, preferably not lower than about 30°C, more preferably not lower than about 50°C. The reaction can be completed generally within 48 hours, preferably within 24 hours, more preferably within 12 hours.

In the above bromination reaction, the rate of configuration inversion is generally not less than 90%, preferably not less than 95%, more preferably not less than 97%. The rate of configuration inversion so referred to herein is expressed in terms of the ratio of the enantiomer excess (% ee) of the product to the enantiomer excess (% ee) of the starting material having the reversed configuration.

The unreacted starting material (optically active 2-sulfonyloxycarboxylic acid (1)) and byproducts (e.g. optically active 2-hydroxycarboxylic acid (4) etc.) generally remain in the reaction mixture after the above bromination reaction. However, the unreacted starting material and byproducts can be efficiently removed by the operations to be mentioned below, namely extraction, washing and crystallization operations.

The desired product, namely optically active 2-bromocarboxylic acid (2), can be separated by an ordinary after-treatment process. For example, the mixture after completion of the reaction can be subjected, if necessary after concentration, to extraction and/or washing using a conventional extraction solvent and water.

As the extraction solvent, there may be mentioned, for example, aliphatic hydrocarbons (preferably aliphatic hydrocarbons containing 5 to 12 carbon atoms, more preferably 5 to 8 carbon atoms), aromatic hydrocarbons (preferably aromatic hydrocarbons containing 6 to 12 carbon atoms, more preferably 6 to 10 carbon atoms, still more preferably 6 to 8 carbon atoms), halogenated hydrocarbons (preferably halogenated hydrocarbons (especially chlorinated hydrocarbons) containing 1 to 6 carbon atoms, more preferably 1 to 4 carbon atoms), esters (preferably esters containing 2 to 8 carbon atoms, more preferably 3 to 5 carbon atoms), ethers (especially acylic ethers (preferably ethers containing 4 to 12 carbon atoms, more preferably 4 to 8 carbon atoms)) and the like.

When an aromatic hydrocarbon is used as the extraction solvent, the desired product (optically active 2-bromocarboxylic acid (2)) alone can be efficiently extracted while the unreacted starting material (optically active 2-sulfonyloxycarboxylic acid (1)) and decomposition products derived therefrom (e.g. optically active 2-hydroxycarboxylic acid (4) etc.) can be eliminated into the aqueous phase. The aromatic hydrocarbon is preferably an aromatic hydrocarbon containing 6 to 12 carbon atoms, more preferably an aromatic hydrocarbon containing 6 to 10 carbon atoms. In particular, aromatic hydrocarbons containing 6 to 8 carbon atoms are preferred and, as specific examples, there may be mentioned benzene, toluene, o-xylene, m-xylene, p-xylene, ethylbenzene, and the like. Among these, toluene is most preferred.

The desired product can be recovered from the extract obtained in the above manner by distilling off the reaction solvent and/or extraction solvent by such as operation as heating under reduced pressure. Although the thus-obtained desired product is nearly pure, its purity may be further increased by such a conventional technique as purification by distillation or column chromatography.

For increasing the chemical purity and/or optical purity, in particular the optical purity, of the optically active 2-bromocarboxylic acid (2) in isolating and purifying the same, the optically active 2-bromocarboxylic acid (2) is preferably crystallized and isolated in the form of a salt with a base.

By crystallizing the acid (2) as a salt with a base, it becomes possible to eliminate those impurities which are otherwise difficult to remove, for example the optical isomer already coexisting and/or formed as a byproduct in small amounts in the production process and/or the corresponding α,β-unsaturated carboxylic acid, and thus obtain the optically active 2-bromocarboxylic acid (2) in a state highly pure chemically as well as optically. The content of the optical isomer as an impurity is not particularly restricted but, from the viewpoint of purification efficiency and the like, it is generally about 1 to 20% by weight, preferably about 1 to 10% by weight, more preferably about 1 to 5% by weight.

The salt of the optically active 2-bromocarboxylic acid (2) with a base is not particularly restricted but includes, for example, metal salts such as alkali metal salts and alkaline earth metal salts; amine salts such as alkylamine salts, aralkylamine salts, arylamine salts, amino acid ester salts and amino acid amide salts; ammonium salts; etc.

More specifically, the salt includes, for example, the lithium salt, sodium salt, potassium salt, magnesium salt, calcium salt, cyclohexylamine salt, dicyclohexylamine salt, 1-phenylethylamine salt, 1-(1-naphthyl)ethylamine salt, benzylamine salt, aniline salt, phenylalanine methyl ester salt, phenylalanine ethyl ester salt, phenylglycine methyl ester salt, phenylalaninamide salt, phenylglycinamide salt, ammonium salt and the like salt of the optically active 2-bromocarboxylic acid (2).

Preferred among them are alkali metal salts such as lithium salt, alkylamine salts such as cyclohexylamine salt and dicyclohexylamine salt, aralkylamine salts such as 1-phenylethylamine salt, amino acid ester salts such as phenylalanine methyl ester salt, phenylalanine ethyl ester salt and phenylglycine methyl ester salt, and amino acid amide salts such as phenylalaninamide salt and phenylglycinamide salt.

The base to be used for converting the optically active 2-bromocarboxylic acid (2) to the corresponding base salt is not particularly restricted but includes, as preferred species, an alkali metal hydroxide, an alkali metal carbonate, an alkali metal hydrogencarbonate, an alkaline earth metal hydroxide, an alkaline earth metal carbonate, an alkylamine, an aralkylamine, an arylamine, an amino acid ester, an amino acid amide, and ammonia, for instance.

More specifically, there may be mentioned, for example, sodium hydroxide, lithium hydroxide, potassium hydroxide, potassium carbonate, lithium carbonate, sodium carbonate, sodium hydrogencarbonate, potassium hydrogencarbonate, magnesium hydroxide, calcium hydroxide, magnesium carbonate, calcium carbonate, cyclohexylamine, dicyclohexylamine, 1-phenylethylamine, 1-(1-naphthyl)ethylamine, benzylamine, aniline, phenylalanine methyl ester, phenylalanine ethyl ester, phenylglycine methyl ester, phenylalaninamide, phenylglycinamide, ammonia, etc.

Preferred among them are alkali metal hydroxides such as lithium hydroxide, alkylamines such as cyclohexylamine and dicyclohexylamine, aralkylamines such as 1-phenylethylamine, amino acid esters such as phenylalanine methyl ester, phenylalanine ethyl ester and phenylglycine methyl ester, and amino acid amides such as phenylalaninamide and phenylglycinamide.

The optically active 2-bromocarboxylic acid (2) can be converted to its salt with a base by mixing the optically active 2-bromocarboxylic acid (2) with the base. The conversion can be adequately carried out using the base in an amount of 1 to 2 equivalents, preferably 0.8 to 1.4 equivalents, more preferably 0.8 to 1.2 equivalents, relative to the optically active 2-bromocarboxylic acid (2). An amount of 0.8 to 1.0 equivalent is especially preferred. The base may be added in the form of a solid or an aqueous solution. Preferably, however, it is added in the form of an aqueous solution.

The salt of the optically active 2-bromocarboxylic acid (2) with a base can be crystallized by mixing the optically active 2-bromocarboxylic acid (2) with the base. Further, the conventional methods of crystallization such as, for example, the cooling crystallization method, the concentration crystallization method, the crystallization method utilizing solvent substitution, the crystallization method by admixing of a poor solvent, and the salting out method may be employed singly or in proper combination. In this case of crystallization, seed crystals may be added according to need.

The above method of crystallization is generally carried out in the presence of a solvent. The solvent is not particularly restricted but includes, for example, aliphatic acid esters, ketones, alcohols, ethers, hydrocarbons, water, etc. More specifically, there may be mentioned, for example, ethyl acetate, isopropyl acetate, acetone, methanol, ethanol, isopropanol, tetrahydrofuran, dioxane, tert-butyl methyl ether, methylene chloride, hexane, heptane, toluene, water, etc.

In the case of crystallization of the optically active 2-bromocarboxylic acid (2) in the above-mentioned metal salt or ammonium salt form, the crystallization is preferably carried out in the presence of water. More specifically and more preferably, the optically active 2-bromocarboxylic acid (2) is mixed with the alkali metal hydroxide, alkali metal carbonate, alkali metal hydrogencarbonate, alkaline earth metal hydroxide, alkaline earth metal carbonate, ammonia or the like in the presence of water to thereby cause the acid (2) to crystallize out from the system in the metal salt or ammonium salt form, still more preferably in the alkali metal form, especially preferably in the lithium salt form. Therefore, the base to be used is preferably an alkali metal hydroxide, most preferably lithium hydroxide.

Generally, the crystallization of the metal salt or ammonium salt of the optically active 2-bromocarboxylic acid (2) is preferably carried out under weakly acidic to basic conditions. When the conditions are expressed in terms of pH, the crystallization can be adequately carried out at pH 4 to 12, preferably at pH 4 to 9, more preferably at pH 4 to 7, still more preferably at pH 4 to 5. At pH below 4, the yield of the above base salt tends to decrease. Since the optically active 2-bromocarboxylic acid (2) has a drawback in that it is unstable under strongly basic conditions in some instances, it is desirable that such strongly basic conditions as pH 14 or above or, further, pH 13 or above, be avoided.

In the crystallization of the above metal salt or ammonium salt, a salt for salting out, an organic solvent and/or the like is preferably used in combination for increasing the yield in an amount which will not produce any adverse effect.

The salt for salting out is not particularly restricted but includes, for example, alkali metal salts such as sodium chloride, potassium chloride, lithium chloride, sodium sulfate, potassium sulfate and lithium sulfate; ammonium salts such as ammonium sulfate and ammonium chloride; alkaline earth metal salts such as calcium chloride; etc. Among them, alkali metal salts are preferred.

The above salt may be added to the system, or may be formed in the system utilizing the acid-base neutralization reaction. The cation of the salt to be used for salting out is preferably the same as the cation in the salt of the optically active 2-bromocarboxylic acid (2) with a base. For example, in the case of salting out of the optically active 2-bromocarboxylic acid (2) in a lithium salt form, a lithium salt, such as lithium chloride or lithium sulfate, is preferably used as the salt to be used for salting out.

The amount of the salt to be used for salting out may vary depending on the salt species, the species of the salt of the optically active 2-bromocarboxylic acid (2) with a base and the amount thereof, hence cannot be specified absolutely. Preferably, however, the salt is used in an amount sufficient to cause salting out in a good yield. More specifically, in the case of crystallization in the presence of water, the salt is preferably caused to exist at a level of about 5% by weight or higher, as expressed in terms of inorganic salt concentration in water, for instance. The upper limit is generally the saturated concentration. In the case of lithium chloride, for instance, the salting out can be.generally carried out favorably within the concentration range of from about 5% by weight to the saturated concentration.

As for the salting out operation, the method comprising adding the salt for salting out prior to salt formation from the optically active 2-bromocarboxylic acid (2) and the base mentioned above, and the method comprising adding the salt for salting out after salt formation from the optically active 2-bromocarboxylic acid (2) and the base mentioned above can be mentioned. The method comprising adding the salt for salting out after salt formation is more preferred. Although the salt for salting out may be added in the form a solid or an aqueous solution, the addition in the form of an aqueous solution is preferred.

When the optically active 2-bromocarboxylic acid (2) is to be crystallized out in the form of a metal salt or ammonium salt in the presence of water, the temperature for this crystallization is not particularly restricted but the crystallization can be favorably carried out generally at 0 to 100°C, for instance. For increasing the impurity removal percentage on the occasion of crystallization, however, it is desirable that the crystallization be carried out at a high temperature, more specifically, preferably at 30°C or above, more preferably at 40°C or above.

The organic solvent to be combinedly used in crystallizing the optically active 2-bromocarboxylic acid (2) in the form of a metal salt or ammonium salt in the presence of water is not particularly restricted but includes, for example, aliphatic hydrocarbons (preferably aliphatic hydrocarbons containing 5 to 12 carbon atoms, more preferably 5 to 8 carbon atoms) such as pentane, hexane and heptane; aromatic hydrocarbons (preferably aromatic hydrocarbons containing 6 to 12 carbon atoms, more preferably 6 to 10 carbon atoms, still more preferably 6 to 8 carbon atoms) such as benzene, toluene, o-xylene, m-xylene, p-xylene and ethylbenzene; halogenated hydrocarbons (preferably halogenated hydrocarbons (in particular chlorinated hydrocarbons) containing 1 to 6 carbon atoms, more preferably 1 to 4 carbon atoms) such as methylene chloride; esters (preferably esters containing 2 to 8 carbon atoms, more preferably 3 to 5 carbon atoms) such as ethyl acetate, isopropyl acetate and butyl acetate; ethers such as tert-butyl methyl ether, diethyl ether, diisopropyl ether, dibutyl ether, tetrahydrofuran, dioxane, 1,2-dimethoxyethane, diethylene glycol dimethyl

ether, triethylene glycol dimethyl ether, tetraethylene glycol dimethyl ether and polyethylene glycol dimethyl ether; alcohols such as methanol, ethanol and 2-propanol; ketones such as acetone and ethyl methyl ketone; nitriles such as acetonitrile; etc.

The organic solvents mentioned above include organic solvents low in compatibility with water, and organic solvents highly compatible with water. The organic solvents low in compatibility with water are not particularly restricted but include, for example, aliphatic hydrocarbons (preferably aliphatic hydrocarbons containing 5 to 12 carbon atoms, more preferably 5 to 8 carbon atoms), aromatic hydrocarbons (preferably aromatic hydrocarbons containing 6 to 12 carbon atoms, more preferably 6 to 10 carbon atoms, still more preferably 6 to 8 carbon atoms), halogenated hydrocarbons (preferably halogenated hydrocarbons (especially chlorinated hydrocarbons) containing 1 to 6 carbons atoms, more preferably 1 to 4 carbon atoms), esters (preferably esters containing 2 to 8 carbon atoms, more preferably 3 to 5 carbon atoms), ethers (in particular acyclic ethers (preferably ethers containing 4 to 12 carbon atoms, more preferably 4 to 8 carbon atoms), and like conventional organic solvents. Among these, the above-mentioned aromatic hydrocarbons are preferred, aromatic hydrocarbons containing 6 to 12 carbon atoms are more preferred, aromatic hydrocarbons containing 6 to 8 carbon atoms are still more preferred, and toluene is most preferred in view of its ease in handling, inexpensiveness, etc.

As the organic solvents highly compatible with water, there may be mentioned such alcohols, ketones and nitriles as mentioned above, etc. However, the above-mentioned ketones are preferred, and acetone is most preferred.

The use of such an organic solvent contributes to the dissolution and elimination of coexisting impurities and/or to the improvement in crystallization yield. The organic solvent may be caused to exist prior to crystallization or may be added after sufficient progress of crystallization. The use of an organic solvent low in compatibility with water among the organic solvents mentioned above results in crystallization in a two-phase system and thus allows the mother liquor after solid-liquid separation to separate into two phases, facilitating the recovery and reuse of the organic solvent, hence is favorable not only from the yield improvement viewpoint but also from the economic viewpoint.

As the most preferred mode of crystallization of the optically active 2-bromocarboxylic acid (2) in the form of a metal salt or ammonium salt, there may be mentioned the method which comprises adding, if necessary, water to a solution of the optically active 2-bromocarboxylic acid (2) in the organic solvent low in compatibility with water (preferably such an aromatic hydrocarbon as mentioned above, in particular toluene) , adding thereto the base (preferably an alkali metal hydroxide, in particular lithium hydroxide) for forming the metal salt or ammonium salt, adjusting the pH to 4 to 5 to thereby cause precipitation of the metal salt or ammonium salt of the optically active 2-bromocarboxylic acid (2), and adding a salt for salting out (preferably an alkali metal salt, in particular lithium chloride or lithium sulfate) to thereby cause further crystallization.

The amount, relative to the optically active 2-bromocarboxylic acid (2), of the organic solvent low in compatibility with water on the occasion of crystallization is not particularly restricted but, from the viewpoint of productivity, crystallization yield, impurity elimination, etc., the weight of the solvent is preferably not more than 4 times, more preferably not more than 3 times, still more preferably not more than 2 times, the weight of the optically active 2-bromocarboxylic acid (2). The amount of water relative to the optically active 2-bromocarboxylic acid (2) on the occasion of crystallization is not particularly restricted but, from the viewpoint of productivity and/or crystallization yield, the weight of water is preferably not more than 20 times, more preferably not more than 10 times, still more preferably not more than 5 times, the weight of the optically active 2-bromocarboxylic acid (2).

The crystals of the metal salt or ammonium salt of the optically active 2-bromocarboxylic acid (2) as obtained in accordance with the invention can be recovered by such a conventional solid-liquid separation method as centrifugation, pressure filtration or vacuum filtration. In collecting the crystals, the crystallization yield can be maximized by cooling the crystallization liquid finally to 20°C or below. The crystals obtained are further subjected to, for example, reduced pressure (vacuum) drying, if necessary, to give dry crystals.

In cases where the optically active 2-bromocarboxylic acid (2) is crystallized as such an amine salt or ammonium salt as mentioned above, the crystallization is preferably carried out in the presence of an organic solvent. More specifically and more preferably, the optically active 2-bromocarboxylic acid (2) is mixed with the amine, ammonia or the like in the presence of an organic solvent to give the amine salt or ammonium salt, which is allowed to crystallize out from the system. Preferred among others are alkylamine salts such as cyclohexylamine salt and dicyclohexylamine salt; aralkylamine salts such as 1-phenylethylamine salt; amino acid ester salts such as phenylalanine methyl ester salt, phenylalanine ethyl ester salt and phenylglycine methyl ester salt; and amino acid amide salts such as phenylalaninamide salt and phenylglycinamide salt. In particular, salts with an amine containing a phenyl group are preferred, and salts with an aralkylamine, an amino acid ester containing a phenyl group, and an amino acid amide containing a phenyl group are most preferred. Specific examples are 1-phenylethylamine salt, phenylalanine methyl ester salt, phenylglycine methyl ester salt, phenylalaninamide salt, and phenylglycinamide salt.

When, as mentioned above, the optically active 2-bromocarboxylic acid (2) is crystallized as the above-mentioned amine salt or ammonium salt, the base to be used is preferably an alkylamine such as cyclohexylamine or dicyclohexylamine; an aralkylamine such as 1-phenylethylamine; an amino acid ester such as phenylalanine methyl

ester, phenylalanine ethyl ester or phenylglycine methyl ester; or an amino acid amide such as phenylalaninamide or phenylglycinamide. Among them, an amine containing a phenyl group is preferred and, in particular, an aralkylamine, an amino acid ester containing a phenyl group, and an amino acid amide containing a phenyl group are preferred. Specifically, there may be mentioned 1-phenylethylamine, phenylalanine methyl ester, phenylglycine methyl ester, phenylalaninamide, and phenylglycinamide.

In cases where the optically active 2-bromocarboxylic acid (2) is crystallized in the form of a salt with an optically active amine, the enantiomer combination of the carboxylic acid and the amine is not particularly restricted. The enantiomer combination can be selected taking into consideration the crystallization yield, impurity elimination, economy, etc. When (R) -2-bromo-3-phenylpropionic acid is crystallized in the form of a salt with 1-phenylethylamine, for example, the (S) form is preferably used as 1-phenylethylamine from the viewpoint of optical purity improvement.

Of course, the above-mentioned base is used fundamentally in an amount of not less than about 1 equivalent relative to the optically active 2-bromocarboxylic acid (2).

In crystallizing the optically active 2-bromocarboxylic acid (2) in the form of an amine salt, the organic solvent to be used is the same as mentioned above. Thus, the aliphatic hydrocarbons, aromatic hydrocarbons, halogenated hydrocarbons, esters, ethers, etc. mentioned hereinabove can be used. In particular, an aprotic organic solvent is preferred, and acetone, methylene chloride, ethyl acetate and toluene are preferred, among others.

In the case of crystallization of the optically active 2-bromocarboxylic acid (2) in the form of an amine salt or ammonium salt in the presence of an organic solvent, the crystallization temperature is not particularly restricted but generally is not higher than 50°C, preferably not higher than 30°C, more preferably not higher than 10°C. Generally, the yields tends to increase as the temperature lowers. Generally, the lower limit to that temperature is not lower than the solidification temperature of the system, for example not lower than -20°C, preferably not lower than 0°C.

Preferred as the optically active 2-bromocarboxylic acid (2) to be crystallized in accordance with the invention are those in which $R^1$ is an aralkyl group containing 7 to 15 carbon atoms, which may optionally be substituted, in particular a benzyl group (i.e. optically active 2-bromo-3-phenylpropionic acid). The optically active 2-bromocarboxylic acid (2) to be used in the above crystallization may be not only one prepared by the process of the present invention but also one prepared by a process so far known in the art (process comprising brominating an optically active amino acid using sodium nitrite while retaining the configuration thereof).

The salt of the optically active 2-bromocarboxylic acid (2) with a base as precipitated out by the above-mentioned method of crystallization can be recovered as crystals, for example, by such a separation operation as filtration. The crystals of the salt of the optically active 2-bromocarboxylic acid (2) with a base can be further purified by washing with water (e.g. cold water, water containing the salt utilized in salting out, etc.) or the above-mentioned organic solvent, for instance.

The thus-obtained salt of the optically active 2-bromocarboxylic acid (2) with a base can be converted to the free optically-active 2-bromocarboxylic acid (2) by neutralization with an acid higher in acidity than the 2-bromocarboxylic acid, for example an inorganic acid such as hydrochloric acid or sulfuric acid, etc. This free-form optically active 2-bromocarboxylic acid (2) can be extracted with an organic solvent, if necessary followed by removal of the solvent, to give the optically active 2-bromocarboxylic acid (2) or a solution thereof.

The above-mentioned organic solvent is not particularly restricted but includes aliphatic hydrocarbons (preferably aliphatic hydrocarbons containing 5 to 12 carbon atoms, more preferably 5 to 8 carbon atoms), aromatic hydrocarbons (preferably aromatic hydrocarbons containing 6 to 12 carbon atoms, more preferably 6 to 10 carbon atoms, still more preferably 6 to 8 carbon atoms), halogenated hydrocarbons (preferably halogenated hydrocarbons (in particular chlorinated hydrocarbons) containing 1 to 6 carbon atoms, more preferably 1 to 4 carbon atoms), esters (preferably esters containing 2 to 8 carbon atoms, more preferably 3 to 5 carbon atoms) , ethers (in particular acyclic ethers (preferably ethers containing 4 to 12 carbon atoms, more preferably 4 to 8 carbon atoms), and like conventional organic solvents. When an aromatic hydrocarbon, for example, is used as the extraction solvent, the desired product, namely the optically active 2-bromocarboxylic acid (2) alone can be efficiently extracted while the optically active 2-sulfonyloxycarboxylic acid (1), the optically active 2-hydroxycarboxylic acid (4), etc., can be eliminated into the aqueous phase.

The aromatic hydrocarbon is preferably an aromatic hydrocarbon containing 6 to 12 carbon atoms, more preferably an aromatic hydrocarbon containing 6 to 10 carbon atoms, still more preferably an aromatic hydrocarbon containing 6 to 8 carbon atoms and, as specific examples, there may be mentioned benzene, toluene, o-xylene, m-xylene, p-xylene, ethylbenzene, etc. Among these, toluene is most preferred.

It is a matter of course that the above-mentioned neutralization (salt interchange) with an acid may be carried out in the presence of the above-mentioned organic solvent.

In cases where the salt of the optically active 2-bromocarboxylic acid (2) with a base is an amine salt, in particular an expensive optically active amine salt, such as the salt with 1-phenylethylamine, 1-(1-naphthyl)ethylamine, an amino acid ester or an amino acid amide, it is desirable that the amine is recovered in the free form by a conventional method for reuse thereof. More specifically, the salt of the amine with an acid as obtained by carrying out the above-mentioned operation with the amine salt of the optically active 2-bromocarboxylic acid (2) can be recovered by solid-liquid separation or extraction with water, and then neutralized with a base in a mixed solvent composed of water and an organic solvent or in an organic solvent to give the free-form amine, which can be extracted into the organic layer. If necessary, the solvent is removed. Thus, the free amine or a solution thereof can be obtained.

The thus-obtained optically active 2-bromocarboxylic acid (2), either in the free form or in the form of a salt, has a high level of purity, namely an optical purity of not lower than 97% ee, preferably not lower than 98% ee, more preferably not lower than 99% ee. The above-mentioned optically active 2-bromocarboxylic acid (2), either in the free form or in the form of a salt, may be the (R) form or the (S) form.

As is obvious from the above description and the description in the examples to be mentioned later herein, the process of the invention makes it possible to obtain the optically active 2-sulfonyloxycarboxylic acid (1), optically active 2-bromocarboxylic acid (2) (inclusive of salts thereof), and optically active 2-hydroxycarboxylic acid ester (5) in an advantageous manner.

BRIEF DESCRIPTION OF THE DRAWINGS

Fig. 1 is a graphic representation of the correlation between the pH and the percent elimination of 2-hydroxy-3-phenylpropionic acid on the occasion of extraction and/or washing in a mixed solvent system comprising a mixed solution composed of toluene or ethyl acetate and water.

Fig. 2 is a graphic representation of relationship between the solubility of lithium (R)-2-bromo-3-phenylpropionate and the lithium chloride concentration.

BEST MODE FOR CARRYING OUT THE INVENTION

The following examples illustrate the present invention in more detail. They are, however, by no means limitative of the scope of the invention. The quantitation of 2-bromo-3-phenylpropionic acid, 2-methanesulfonyloxy-3-phenylpropionic acid, 2-hydroxy-3-phenylpropionic acid and cinnamic acid was carried out using the following analytical system.

[Column: Nomura Chemical's Develosil ODS-HG-3, 150 mm x 4. 6 mm I.D., mobile phase: phosphate buffer/acetonitrile = 2/1, flow rate: 1.0 ml/min, detection: UV 210 nm, column temperature: 40°C, retention times: 2-hydroxy-3-phenylpropionic acid 2.9 min, 2-methanesulfonyloxy-3-phenylpropionic acid 4.2 min, cinnamic acid 6.3 min, 2-bromo-3-phenylpropionic acid 9.8 min.]

The optical purities of 2-methanesulfonyloxy-3-phenylpropionic acid and 2-bromo-3-phenylpropionic acid were evaluated by the following methods, respectively, following derivatization to the methyl esters.

<Optical purity evaluation of 2-methanesulfonyloxy-3-phenylpropionic acid>

The product (20 mg, 0.08 mmol) was dissolved in 1 ml of methanol, 166 mg (0.15 mmol) of a 10% trimethylsilyldiazomethane solution was added dropwise, the reaction was allowed to proceed at room temperature for 30 minutes, the solvent was then distilled off under reduced pressure, and the concentrate was purified on a silica gel column (hexane/ethyl acetate = 4/1) to give methyl 2-methanesulfonyloxy-3-phenylpropionate. This methyl ester was analyzed under the following HPLC conditions.
Column: Daicel Chemical Industries' Chiralcel OD-H, 250 mm x 4.6 mm I.D.
Mobile phase: hexane/2-propanol = 90/10
Flow rate: 1.0 ml/min
Detection: UV 210 nm
Column temperature: 40°C
Retention time: S-form 13.4 min, R-form 11.7 min

<Optical purity evaluation of 2-bromo-3-phenylpropionic acid>

The product (20 mg, 0.09 mmol) was dissolved in 1 ml of methanol, 166 mg (0.15 mmol) of a 10% trimethylsilyldiazomethane solution was added dropwise, the reaction was allowed to proceed at room temperature for 30 minutes, the solvent was then distilled off under reduced pressure, and the concentrate was purified on a silica gel column (hexane/ethyl acetate = 5/1) to give methyl 2-bromo-3-phenylpropionate. This methyl ester was analyzed under the following HPLC conditions.
Column: Daicel Chemical Industries' Chiralpak AD, 250 mm x 4.6 mm I.D., and Precolumn exclusively for Chiralpak AD, 50 mm x 4.6 mm I.D.

Mobile phase: hexane
Flow rate: 1.0 ml/min
Detection: UV 210 nm
Column temperature: 25°C
Retention time: S-form 26.9 min, R-form 20.7 min

(Reference Example 1) (S)-2-Hydroxy-3-phenylpropionic acid

L-Phenylalanine (400 g) was added to a solution prepared by diluting 237 g of concentrated sulfuric acid with 4400 g of water and, then, a mixture of 418 g of sodium nitrite and 800 g of water was added over 5 hours at an inside temperature of 20°C. After the addition, the mixture was continuously stirred at 20°C for 20 hours. Thereafter, 4000 ml of tert-butyl methyl ether was added and, after 30 minutes of stirring at 20°C, the organic layer was separated (extract 1). Further, 2000 ml of tert-butyl methyl ether was added to the aqueous layer and, after 30 minutes of stirring at 20°C, the organic layer was separated (extract 2). The extract 1 and extract 2 were combined. The whole extract (4644 g) contained 348.7 g of (S)-2-hydroxy-3-phenylpropionic acid. This extract was concentrated under reduced pressure to give 1392 g of a concentrate. Hexane (4200 ml) was added gradually to this concentrate with stirring at 40°C for causing crystallization, and the mixture was then cooled to 5°C and continuously stirred for 2 hours. The crystals thus formed were collected by filtration under reduced pressure and then washed with two 680-ml portions of hexane/tert-butyl methyl ether (75/25 by volume). The wet crystals obtained were subjected to reduced pressure (vacuum) drying (full vacuum, 40°C, overnight) to give 312 g of (S)-2-hydroxy-3-phenylpropionic acid (purity 98.7%, yield 77%).

(Reference Example 2) (R)-2-Bromo-3-phenylpropionic acid

D-Phenylalanine (100 g) was added to a solution prepared by diluting 150 g of sulfuric acid with 1100 g of water and, then, 360 g of potassium bromide was added at an inside temperature of 20°C. The mixture was cooled to an inside temperature of -10°C and, in succession, a mixed solution composed of 64 g of sodium nitrite and 120 g of water was added over 2 hours. After completion of the addition, the mixture was stirred at -10°C for 3.5 hours and 1000 ml of toluene was then added, and the mixture was stirred at 20°C for 30 minutes. Thereafter, the organic layer was separated (extract 1). Further, 100 ml of toluene was added to the aqueous layer and, after 30 minutes of stirring at 20°C, the organic layer was separated (extract 2). The extracts 1 and 2 were combined. The whole extract (1086 g) contained 112 g of (R)-2-bromo-3-phenylpropionic acid. This extract was washed with six 200-ml portions of water to give 1063 g of an organic layer. This organic layer was concentrated to give 124.7 g of an oily (R)-2-bromo-3-phenylpropionic acid ((R)-2-bromo-3-phenylpropionic acid 109 g (optical purity 94.0% ee, yield 78%, cinnamic acid content 3.71 g).

(Reference Example 3) (R)-2-Bromo-3-phenylpropionic acid

D-Phenylalanine (500 g) was added to a solution prepared by diluting 2040 g of 47% hydrobromic acid with 750 g of water at an inside temperature of 0°C, the mixture was cooled to an inside temperature of -5°C and, in succession, a mixed solution composed of 272 g of sodium nitrite and 510 g of water over 5 hours. After completion of the addition, the mixture was stirred at an inside temperature of -5°C for 3 hours and then, after raising the temperature to 20°C, the mixture was stirred for 1 hour. Toluene (1600 ml) was added to this reaction mixture and, after 30 minutes of stirring at 20°C, the organic layer was separated (extract 1). Further, 800 ml of toluene was added to the aqueous layer and, after 30 minutes of stirring at 20°C, the organic layer was separated (extract 2). The extracts 1 and 2 were combined, and the whole extract was washed with four 500-ml portions of water (extract 3). The extract 3 obtained was concentrated under reduced pressure to give 1509 g of a concentrate. This concentrate contained 603 g of (R)-2-bromo-3-phenylpropionic acid (optical purity 94.3% ee, yield 87%, cinnamic acid content 3.32 g).

(Example 1) Methyl (S)-2-hydroxy-3-phenylpropionate

Thionyl chloride (249.1 g, 2.09 mol) was added to 558.4 g (17.45 mol) of methanol at an inside temperature of 10°C over 2 hours, and the mixture was stirred at an inside temperature of 10°C for 1 hour. To this solution was added a mixed solution composed of 289.0 g (1.739 mol) of (S)-2-hydroxy-3-phenylpropionic acid and 558.4 g of methanol over 2 hours at an inside temperature of 10°C. The mixture was continuously stirred at 5°C for 1 hour, 650 g of pure water was then added slowly at 10°C and, further, 1156 ml of toluene was added, and the mixture was stirred for 30 minutes. In succession, 857.8 g of a 30% aqueous sodium hydroxide solution was added at an inside temperature of 10°C until a pH of 5.0 and, after 30 minutes of stirring at 10°C, the organic layer was separated (extract 1). Further,

578 ml of toluene was added to the aqueous layer and, after 30 minutes of stirring at 10°C, the organic layer was separated (extract 2). The extracts 1 and 2 were combined. Pure water (289 ml) was added to the whole extract (1890 g), the pH was adjusted to 8 to 9 by adding a 30% aqueous sodium hydroxide solution at 10°C and, after 30 minutes of stirring at 10°C, the organic layer was separated. The organic layer obtained was washed with two 289-ml portions of pure water (extract 3). The extract 3 obtained was concentrated under reduced pressure to give 1597 g of a concentrate. This contained 268.5 g of methyl (S) -2-hydroxy-3-phenylpropionoate (yield 86%, optical purity 99.8% ee).

(Example 2) Methyl (S)-2-hydroxy-3-phenylpropionate

Methanesulfonic acid (3.36 g, 0.036 mol) was added to a solution composed of 120 g (0.722 mol) of (S)-2-hydroxy-3-phenylpropionic acid and 1200 ml of methanol, and the mixture was stirred at an inside temperature of 55°C for 24 hours and then cooled to 20°C (2-hydroxy-3-phenylpropionic acid content: 1.0%). Pure water (100 g) was added to the reaction mixture, and the pH was adjusted to 4.5 by adding 54.6 g of a 5% aqueous sodium hydrogencarbonate solution at 10°C. The resulting solution was concentrated to 290 g under reduced pressure, 1000 ml of toluene was then added and, after 30 minutes of stirring at 10°C, the organic layer was separated (extract 1). Further, 500 ml of toluene was added to the aqueous layer and, after 30 minutes of stirring at 10°C, the organic layer was separated (extract 2). The extracts 1 and 2 were combined. Pure water (200 ml) was added to the whole extract, the pH was adjusted to 6.2 by adding 10.7 g of a 5% aqueous sodium hydrogencarbonate solution at 10°C, and the organic layer was then separated. The organic layer obtained was further washed with 200 ml of pure water and then concentrated under reduced pressure to give 1258 g of a concentrate. This contained 124.2 g of methyl (S)-2-hydroxy-3-phenylpropionate (yield 97%, optical purity 99.8% ee, 2-hydroxy-3-phenylpropionic acid: not detected).

(Example 3) Methyl (S)-2-hydroxy-3-phenylpropionate

Methanesulfonic acid (0.14 g) was added to a solution composed of 5 g (30.1 mmol) of (S) -2-hydroxy-2-phenylpropionic acid and 50 ml of methanol, and the mixture was stirred at an inside temperature of 58°C for 24 hours and then cooled to 20°C (2-hydroxy-3-phenylpropionic acid content: 1.1%). Pure water (5 g) was added to the reaction mixture, and the pH was adjusted to 4.6 by adding 2.7 g of a 5% aqueous sodium hydrogencarbonate solution at 10°C. The resulting solution was concentrated to about 36 g under reduced pressure, 50 ml of ethyl acetate was then added and, after 30 minutes of stirring at 10°C, the organic layer was separated (extract 1). Further, 20 ml of ethyl acetate was added to the aqueous layer and, after 30 minutes of stirring at 10°C, the organic layer was separated (extract 2). The extracts 1 and 2 were combined. Pure water (10 ml) was added to the whole extract., the pH was adjusted to 7.1 by adding 0.4 g of a 5% aqueous sodium hydrogencarbonate solution at 10°C, and the organic layer was then separated. The organic layer obtained was further washed with 200 ml of pure water (pH 4.9) to give 71.4 g of an organic layer. This contained 5.26 g of methyl (S)-2-hydroxy-3-phenylpropionate (yield 97%, optical purity 99.8% ee, 2-hydroxy-3-phenylpropionic acid content: 0.26%).

(Example 4) Methyl (S)-2-hydroxy-3-phenylpropionate

Pure water (5 g) was added to a methyl (S)-2-hydroxycarboxylate-containing reaction mixture separately obtained (2-hydroxycarboxylic acid content: 1.1%), and the pH was adjusted to 4.6 by adding 2.7 g of a 5% aqueous sodium hydrogencarbonate solution at 10°C. The resulting solution was concentrated to about 36 g under reduced pressure, 80 ml of heptane was then added and, after 30 minutes of stirring at 10°C, the organic layer was separated (extract 1). Further, 20 ml of heptane was added to the aqueous layer and, after 30 minutes of stirring at 10°C, the organic layer was separated (extract 2). The extracts 1 and 2 were combined. Pure water (10 ml) was added to the whole extract, the pH was adjusted to 7.1 by adding 0.4 g of a 5% aqueous sodium hydrogencarbonate solution at 10°C, and the organic layer was then separated. The organic layer obtained was further washed with 200 ml of pure water (pH 4.9) to give 100 g of an organic layer. This contained 5.3 g of methyl (S)-2-hydroxy-3-phenylpropionate (yield 97%, optical purity 99.8% ee, 2-hydroxy-3-phenylpropionic acid: not detected).

(Example 5)

A mixed solution was prepared by adding 0.1 g of (S)-2-hydroxy-3-phenylpropionic acid to 100 g of a toluene solution containing 10 g of methyl (S) -2-hydroxy-3-phenylpropionate separately prepared. A 10-g portion was extracted from this solution, 2 ml of 0.1 mol/l hydrochloric acid was added, and the pH of the resulting two-layer mixture was adjusted to each predetermined pH by adding a 5% aqueous sodium hydrogencarbonate solution at 10°C. After further 30 minutes of stirring at 10°C, the organic layer was separated and subjected to HPLC analysis for quantitating 2-hydroxy-3-phenylpropionic acid. The thus-obtained correlation between the percent elimination (formula 1 given below)

of 2-hydroxy-3-phenylpropionic acid by the washing operation and the pH is shown in Fig. 1.

(Formula 1)

Elimination (%) = [{2-hydroxy-3-phenylpropionic acid (g) before washing} - {2-hydroxy-3-phenylpropionic acid (g) after washing}]/{2-hydroxy-3-phenylpropionic acid (g) before washing} x 100.

(Example 6) Methyl (S)-2-hydroxy-3-phenylpropionate

A toluene solution (12.6 g) of (S)-2-hydroxy-3-phenylpropionic acid separately prepared (containing 2.0 g of methyl (S)-2-hydroxy-3-phenylpropionate, optical purity 99.8% ee) was concentrated under reduced pressure to give 2.37 g of a concentrate. To this was added 15 g of heptane at 50 to 55°C for precipitation of crystals (heptane/toluene = 41/1 (weight/weight)). The resulting slurry was cooled to 5°C, stirred at the same temperature for 1 hour, and then filtered. The wet crystals obtained were dried under reduced pressure to give 1.75 g of dry crystals of methyl (S)-2-hydroxy-3-phenylpropionate (yield 88%, optical purity 100.0% ee).

(Example 7) Methyl (S)-2-hydroxy-3-phenylpropionate

An ethyl acetate solution (27.1 g) of (S)-2-hydroxy-3-phenylpropionic acid separately prepared (containing 2.0 g of methyl (S)-2-hydroxy-3-phenylpropionate, optical purity 99.8% ee) was concentrated under reduced pressure to give 2.2 g of a concentrate (75/1 = heptane/ethyl acetate (weight/weight)). To this was added 15 g of heptane at 50 to 55°C for precipitation of crystals. The resulting slurry was cooled to 5°C, stirred at the same temperature for 1 hour, and then filtered. The wet crystals obtained were dried under reduced pressure to give 1.49 g of dry crystals of methyl (S)-2-hydroxy-3-phenylpropionate (yield 75%, optical purity 100.0% ee).

(Example 8) Methyl (S)-2-methanesulfonyloxy-3-phenylpropionate

Triethylamine (300 g, 2.965 mol) was added over 30 minutes to 2668 g of a toluene solution containing 267 g (1.482 mol) of methyl (S)-2-hydroxy-3-phenylpropionate obtained in Example 1 at an inside temperature of 10°C. After 30 minutes of stirring at 10°C, 254.7 g (2.223 mol) of methanesulfonyl chloride was added in succession at 10°C over 4 hours. After 2 hours of stirring at 5°C, 1600 ml of pure water was added and, further, 36 g of concentrated hydrochloric acid was added to adjust the pH to 5 to 6 and, after 30 minutes of stirring at 20°C, the organic layer was separated (extract). This extract was washed with 534 ml of pure water and further concentrated under reduced pressure to give 1826 g of a concentrate. The concentrate obtained contained 366.4 g of methyl (S)-2-methanesulfonyloxy-3-phenylpropionate (yield 96%, optical purity 99.8% ee).

(Example 9) Methyl (S)-2-(4-chlorobenzenesulfonyloxy)-3-phenylpropionate

Triethylamine (11.2 g, 0.110 mol) was added over 30 minutes to a toluene solution (67.1 g) containing 10.0 g (0.055 mol) of methyl (S)-2-hydroxy-3-phenylpropionate separately prepared (optical purity 100% ee) at an inside temperature of 10°C. After 30 minutes of stirring at 10°C, a mixture of 17.6 g (0.083 mol) of 4-chlorobenzenesulfonyl chloride and 50 ml of toluene was added in succession at 10°C over 4 hours. After 15 hours of stirring at 10°C, 50 ml of pure water was added, and the organic layer was separated (extract). This extract was washed with 50 ml of pure water and further concentrated under reduced pressure to give 28.6 g of oily methyl (S)-2-(4-chlorobenzenesulfonyloxy)-3-phenylpropionate.

(Example 10) Methyl (S)-2-methanesulfonyloxypropionate

Triethylamine (40.5 g, 0. 40 mol) was added over 30 minutes to a solution prepared by mixing 20.8 g (0.20 mol) of methyl L-lactate (optical purity 100% ee) with 200 ml of toluene at an inside temperature of 10°C. After 30 minutes of stirring at 10°C, 34.4 g (0.30 mol) ofmethanesulfonyl chloride was added in succession at 10°C over 4 hours. After 2 hours of stirring at 5°C, 100 ml of pure water was added and, further, 36 g of concentrated hydrochloric

acid was added to adjust the pH to 5 to 6 and, after 30 minutes of stirring at 20°C, the organic layer was separated (extract). This extract was washed with 100 ml of pure water and further concentrated under reduced pressure to give 36.2 g of oily methyl (S)-2-methanesulfonyloxypropionate.

(Example 11) (S)-2-Methanesulfonyloxy-3-phenylpropionic acid

The toluene solution (1800 g) obtained in Example 8, which contained 360 g (1.394 mol) of methyl (S)-2-methanesulfonyloxy-3-phenylpropionate, was concentrated under reduced pressure to 720 g. Pure water (720 g), 641.7 g (13.94 mol) of formic acid and 136.7 g (1.394 mol) of sulfuric acid were added to the concentrate at 20°C, and the inside temperature was raised to 70°C. Thereafter, the mixture was stirred in succession at 70°C for 15 hours, and 200 g of the contents were distilled off under reduced pressure. Thereafter, the remaining mixture was stirred at 70°C for 5 hours and, then, 200 g of the contents were again distilled off under reduced pressure. Thereafter, the remaining mixture was stirred at 70°C for 2 hours and then cooled to an inside temperature of 40°C. Thereafter, the contents were concentrated under reduced pressure to 1060 g, and cooled to an inside temperature of 10°C. In succession, 720 ml of pure water and 720 ml of toluene were added, the pH was then adjusted to 11 by adding 1310 g of a 30% aqueous sodium hydroxide solution and, after 30 minutes of stirring at 10°C, the aqueous layer was separated. The aqueous layer separated was washed with 360 ml of toluene. To this aqueous layer was added 720 ml of tert-butyl methyl ether, the pH was adjusted to 1 by adding concentrated hydrochloric acid at 10°C and, after 30 minutes of stirring at 10°C, the organic layer was separated. This organic layer was washed with two 360-ml portions of pure water. The thus-obtained organic layer (830.9 g) contained 285.0 g of (S)-2-methanesulfonyloxy-3-phenylpropionic acid (yield 84%, optical purity 99.8% ee, 2-hydroxy-3-phenylpropionic acid content 8.83 g). The rate of configuration retention in this reaction was 100%.

(Example 12) (S)-2-Methanesulfonyloxy-3-phenylpropionic acid

A toluene solution (70 g) containing 7.0 g (27. 11 mmol) of methyl (S)-2-methanesulfonyloxy-3-phenylpropionate (100% ee), prepared separately, was concentrated under reduced pressure to 14 g. Pure water (14 g) , 35 g of 1,4-dioxane and 2.7 g of sulfuric acid were added to the concentrate at 20°C, and the inside temperature was raised to 90°C. Thereafter, the mixture was stirred in succession at 90°C for 8 hours, and 17 g of the contents were distilled off under reduced pressure. Thereafter, the remaining mixture was further stirred at 90°C for 14 hours, and then cooled to an inside temperature of 10°C. In succession, 28 ml of pure water and 14 ml of toluene were added, the pH was then adjusted to 6 by adding 11 g of a 30% aqueous sodium hydroxide solution and, after 30 minutes of stirring at 10°C, the aqueous layer was separated. The aqueous layer separated was washed with 7 ml of toluene. To this aqueous layer was added 14 ml of tert-butyl methyl ether, the pH was adjusted to 1 by adding concentrated hydrochloric acid at 10°C and, after 30 minutes of stirring at 10°C, the organic layer was separated. This organic layer was washed with two 7-ml portions of pure water. It contained 5.2 g of (S)-2-methanesulfonyloxy-3-phenylpropionic acid (yield 79%, optical purity 100% ee). The rate of configuration retention in this reaction was 100%.

(Example 13) (S)-2-Methanesulfonyloxy-3-phenylpropionic acid

A toluene solution (70 g) containing 7.0 g (27.11 mmol) of methyl (S)-2-methanesulfonyloxy-3-phenylpropionate (100% ee), prepared separately, was concentrated under reduced pressure to 23 g. Pure water (23 g) was added at 20°C, 3.98 g of a 30% aqueous sodium hydroxide solution was added slowly at an inside temperature of 5°C and, after 3 hours of stirring at an inside temperature of 5°C, the aqueous layer was separated. The aqueous layer separated was washed with 7 ml of toluene. To this aqueous layer was added 14 ml of tert-butyl methyl ether, the pH was adjusted to 1 by adding concentrated hydrochloric acid at 10°C and, after 30 minutes of stirring at 10°C, the organic layer was separated. This organic layer was washed with two 7-ml portions of pure water. It contained 6.2 g of (S)-2-methanesulfonyloxy-3-phenylpropionic acid (yield 94%, optical purity 95.2% ee). The rate of configuration retention in this reaction was 95.2%.

(Example 14) (S)-2-Methanesulfonyloxy-3-phenylpropionic acid

Toluene (600 ml) was added to a 225-g portion of the (S)-2-methanesulfonyloxy-3-phenylpropionic acid/tert-butyl methyl ether extract obtained in Example 11, which portion contained 77.2 g of (S)-2-methanesulfonyloxy-3-phenylpropionic acid, optical purity 99.8% ee, 2-hydroxy-3-phenylpropionic acid content 6.97 g), and the mixture was concentrated under reduced pressure to 373 g for precipitation of crystals, then cooled to 5°C, and further stirred for 1 hour. The crystals formed were collected by filtration under reduced pressure and then washed with two 300-ml portions of toluene. The wet crystals obtained were subjected to reduced pressure (vacuum) drying (full vacuum, 40°C,

overnight) to give 73.1 g of (S)-2-methanesulfonyloxy-3-phenylpropionic acid (purity 99%, optical purity 100% ee, crystallization yield 95%, 2-hydroxy-3-phenylpropionic acid content 0.15 g).
$^1$H-NMR ($CDCl_3$) δ (ppm): 2.74 (s, 3H), 3.16 (m, 1H), 3.36 (m, 1H), 5.21 (m, 1H), 7.27-7.37 (m, 5H), 7.53 (bs, 1H).

(Example 15) (S)-2-(4-chlorobenzenesulfonyloxy)-3-phenylpropionic acid

Toluene (9.4 g), 35 g of pure water, 22.3 g (0.48 mol) of formic acid and 4.75 g (0.048 mol) of sulfuric acid were added at 20°C to 25.0 g of the oily product obtained in Example 9, and the inside temperature was raised to 80°C. Thereafter, the mixture was stirred at 80°C for 15 hours and then cooled to an inside temperature of 40°C. After cooling, the contents were concentrated under reduced pressure to 29.0 g and then cooled to an inside temperature of 10°C. In succession, 35 ml of pure water and 35 ml of toluene were added, and the pH was adjusted to 5.5 by adding 20.8 g of a 30% aqueous sodium hydroxide solution to crystallize. The crystals formed were collected by filtration under reduced pressure and then washed with two 25-ml portions of toluene to give 22.9 g of wet crystals. To the wet crystals were added 100 ml of tert-butyl methyl ether and 100 ml of pure water, the pH was adjusted to 1 by adding concentrated hydrochloric acid at 10°C and, after 30 minutes of stirring at 10°C, the organic layer was separated. This organic layer was washed with 50 ml of pure water to give 97.8 g of an (S)-2-(4-chlorobenzenesulfonyloxy)-3-phenylpropionic acid/tert-butyl methyl ether extract. Toluene (150 ml) was added to this extract, and the mixture was concentrated under reduced pressure to 35.2 g for causing crystallization and then cooled to 5°C, followed by 1 hour of stirring. The crystals formed were filtered off under reduced pressure and then washed with two 10-ml portions of toluene. The wet crystals obtained were subjected to reduced pressure (vacuum) drying (full vacuum, 40°C, overnight) to give 5.69 g of (S)-2-(4-chlorobenzenesulfonyloxy)-3-phenylpropionic acid.
$^1$H-NMR ($CDCl_3$) δ (ppm): 3.06 (m, 1H), 3.24 (m, 1H), 4.97 (m, 1H), 6.16 (bs, 1H), 7.05-7.10 (m, 2H), 7.16-7.23 (m, 3H), 7.28-7.30 (m, 2H), 7.50-7.52 (m, 2H).

(Example 16) (S)-2-Methanesulfonyloxypropionic acid

Pure water (70.0 g), 88. 5 g (1.92 mol) of formic acid and 18.9 g (0.19 mol) of sulfuric acid were added to 35.0 g of the oily methyl (S)-2-methanesulfonyloxypropionate obtained in Example 10 at 20°C, and the inside temperature was raised to 70°C. Thereafter, the mixture was stirred at 70°C for 15 hours and then cooled to an inside temperature of 40°C. After cooling, the contents were concentrated to 118 g under reduced pressure and then cooled to an inside temperature of 10°C. In succession, 70 ml of pure water and 70 ml of toluene were added, the pH was adjusted to 11 by adding 193 g of a 30% aqueous sodium hydroxide solution and, after 30 minutes of stirring at 10°C, the aqueous layer was separated. The aqueous layer separated was washed with 35 ml of toluene. To this aqueous layer was added 70 ml of tert-butyl methyl ether, the mixture was adjusted to pH 1 by adding concentrated hydrochloric acid at 10°C and, after 30 minutes of stirring at 10°C, the organic layer was separated. This organic layer was washed with two 35-ml portions of pure water to give 63.2 g of an (S)-2-methanesulfonyloxypropionic acid/tert-butyl methyl ether extract. Toluene (100 ml) was added to this extract, the mixture was concentrated under reduced pressure to 10.3 g to crystallize, then cooled to 5°C, and further stirred for 1 hour. The crystals formed were collected by filtration under reduced pressure and then washed with two 3-ml portions of toluene. The wet crystals obtained were subjected to reduced pressure (vacuum) drying (full vacuum, 35°C, 3 hours) to give 5.18 g of (S)-2-methanesulfonyloxypropionic acid.
$^1$H-NMR ($CDCl_3$) δ (ppm): 1.68 (d, J = 6.8 Hz, 3H), 3.16 (m, 3H), 5.19 (q, J = 6.8 Hz, 1H), 7.96 (bs, 1H).

(Example 17) (R)-2-Bromo-3-phenylpropionic acid

Lithium bromide (1.07 g) was added to a mixture of 1.0 g of (S)-2-methanesulfonyloxy-3-phenylpropionic acid obtained in Example 14 and 20 ml of toluene added thereto, the whole mixture was stirred at 50°C for 5 hours and then cooled to 20°C, 5 ml of pure water was then added and, after 30 minutes of stirring at 20°C, the organic layer was separated. The organic layer obtained contained 0.746 g of (R)-2-bromo-3-phenylpropionic acid (yield 80%, optical purity 96.5% ee). The rate of configuration inversion in this reaction was 96.5%.

(Example 18) (R)-2-Bromo-3-phenylpropionic acid

Lithium bromide (1.07 g) was added to a mixture of 1.0 g of (S)-2-methanesulfonyloxy-3-phenylpropionic acid (100% ee) and 20 ml of methylene chloride added thereto, the whole mixture was refluxed for 18 hours and then cooled to 20°C, 5 ml of pure water was then added and, after 30 minutes of stirring at 20°C, the organic layer was separated. The organic layer obtained contained 0.792 g of (R)-2-bromo-3-phenylpropionic acid (yield 84%, optical purity 96.2% ee). The rate of configuration inversion in this reaction was 96.2%.

(Example 19) (R)-2-Bromo-3-phenylpropionic acid

Lithium bromide (1.07 g). was added to a mixture of 1.0 g of (S)-2-methanesulfonyloxy-3-phenylpropionic acid (100% ee) and 20 ml of diisopropyl ether added thereto, the whole mixture was stirred at 50°C for 6 hours and then cooled to 20°C, 5 ml of pure water was then added and, after 30 minutes of stirring at 20°C, the organic layer was separated. The organic layer obtained contained 0.786 g of (R)-2-bromo-3-phenylpropionic acid (yield 84%, optical purity 95.9% ee). The rate of configuration inversion in this reaction was 95.9%.

(Example 20) (R)-2-Bromo-3-phenylpropionic acid

Lithium bromide (0.36 g) was added to a mixture of 1.0 g of (S)-2-methanesulfonyloxy-3-phenylpropionic acid (95.2% ee) and 10 ml of toluene added thereto, the whole mixture was stirred at 50°C for 24 hours and then cooled to 20°C, 5 ml of pure water was then added and, after 30 minutes of stirring at 20°C, the organic layer was separated. The organic layer obtained contained 0.893 g of (R)-2-bromo-3-phenylpropionic acid (yield 95%, optical purity 94.2% ee). The rate of configuration inversion in this reaction was 98.9%.

(Example 21) (R)-2-Bromo-3-phenylpropionic acid

Toluene (43.3 g) was added to 29.2 g of the solution of (S)-2-methanesulfonyloxy-3-phenylpropionic acid in tert-butyl methyl ether as obtained in Example 11 (10.0 g of (S)-2-methanesulfonyloxy-3-phenylpropionic acid, 99.8% ee, and 0.31 g of 2-hydroxy-3-phenylpropionic acid being contained in the solution), and the same weight as that of the toluene added was removed by concentration under reduced pressure. Further, the same toluene addition and concentration procedure was repeated three times, the temperature was then raised to 60°C and, after 1 hour of stirring, 4.26 g of lithium bromide was added, followed by 6 hours of stirring at 60°C. At this time point, the reaction mixture contained 7.97 g of (R)-2-bromo-3-phenylpropionic acid (yield 85%, 94.8% ee) , 0.72 g of (S)-2-methanesulfonyloxy-3-phenylpropionic acid and 0.31 g of 2-hydroxy-3-phenylpropionic acid. This reaction mixture was cooled to 20°C and, after addition of 250 ml of pure water, the pH was adjusted to 8 by addition of a 30% aqueous sodium hydroxide solution at 5°C, and the aqueous layer was separated. This aqueous layer was washed with three 80-ml portions of toluene, 170 ml of toluene was then added, the pH was adjusted to 1.5 with concentrated hydrochloric acid, whereby a toluene extract was obtained (this toluene extract contained 7.85 g of (R)-2-bromo-3-phenylpropionic acid (94.8% ee), 0.16 g of (S)-2-methanesulfonyloxy-3-phenylpropionic acid, and 0.06 g of 2-hydroxy-3-phenylpropionic acid). This toluene extract was washed with three 50-ml portions of pure water, and the organic layer was separated. The thus-obtained washed extract contained 7.65 g of (R)-2-bromo-3-phenylpropionic acid (yield 82%, 94.8% ee), 0.02 g of (S)-2-methanesulfonyloxy-3-phenylpropionic acid, and 0.01 g of 2-hydroxy-3-phenylpropionic acid. The rate of configuration inversion in this reaction was 95.0%.

(Example 22) (R)-2-Bromo-3-phenylpropionic acid

Lithium bromide (6.42 g) was added to a mixture of 6.0 g of (S)-2-methanesulfonyloxy-3-phenylpropionic acid (100% ee) and 120 ml of methylene chloride added thereto, and the whole mixture was refluxed for 18 hours. At this time point, the reaction mixture contained 4.50 g of (R)-2-bromo-3-phenylpropionic acid (yield 80%, 95.8% ee) and 0.72 g of (S)-2-methanesulfonyloxy-3-phenylpropionic acid. This reaction mixture was cooled to 20°C and, after addition of 150 ml of pure water, the pH was adjusted to 8 by addition of a 30% aqueous sodium hydroxide solution at 5°C, and the aqueous layer was separated. This aqueous layer was washed with three 50-ml portions of toluene, 100 ml of toluene was then added, and the pH was adjusted to 1.5 with concentrated hydrochloric acid to give a toluene extract (this toluene extract contained 4.43 g of (R)-2-bromo-3-phenylpropionic acid (95.8% ee), and 0.16 g of (S)-2-methanesulfonyloxy-3-phenylpropionic acid). This toluene extract was washed with three 50-ml portions of pure water, and the organic layer was separated. The thus-obtained washed extract contained 4.32 g of (R)-2-bromo-3-phenylpropionic acid (yield 77%, 95.8% ee), and 0.02 g of (S)-2-methanesulfonyloxy-3-phenylpropionic acid. The rate of configuration inversion in this reaction was 95.8%.

(Example 23) (R)-2-Bromo-3-phenylpropionic acid

Pure water (300 ml) was added to 2250 g of an (R)-2-bromo-3-phenylpropionic acid/toluene solution (containing 93.7 g of (R)-2-bromo-3-phenylpropionic acid, optical purity 95.0% ee) separately obtained, and the pH was then adjusted to 5.3 by adding 110 g of a 10% aqueous lithium hydroxide solution at an inside temperature of 10°C and, after 30 minutes of stirring at 10°C, the aqueous layer was separated. The aqueous layer obtained was concentrated under reduced pressure to 200 g, 10 L of acetone was added over 1 hour at an inside temperature of 20°C to

thereby crystallize, the mixture was then cooled to 5°C and further stirred for 2 hours. The crystals formed were collected by filtration under reduced pressure and then washed with 300 ml of acetone. The thus-obtained wet crystals were dried under reduced pressure to give 65.0 g of lithium (R)-2-bromo-3-phenylpropionate as dry crystals.
$^1$H-NMR ($D_2O$) δ (ppm): 3.23 (m, 1H), 3.36 (m, 1H), 4.45 (t, J = 7.6 Hz, 1H), 7.21-7.32 (m, 5H).
IR (KBr) : 3536, 1595, 1422, 1341, 1252, 1206, 1142, 1080, 1040, 961, 922, 830, 793, 749, 722, 695, 586, 565, 517 ($cm^{-1}$).

Pure water (500 ml) was added to the dry crystals for dissolution thereof, and the solution was washed with two 500-ml portions of toluene. Toluene (500 ml) was added to the aqueous layer, the pH was adjusted to 1 by adding 28.5 g of concentrated hydrochloric acid at an inside temperature of 10°C, the mixture was stirred at 10°C for 30 minutes and, then, the organic layer was separated. This organic layer was washed with three 300-ml portions of pure water and concentrated under reduced pressure to give 67.4 g of a concentrate, which contained 62.7 g of (R)-2-bromo-3-phenylpropionic acid (yield 67%, optical purity 99.7% ee).
$^1$H-NMR ($CDCl_3$) δ (ppm): 3.25 (m, 1H), 3.47 (m, 1H), 4.42 (t, J = 7.3 Hz, 1H), 7.21-7.32 (m, 5H), 9.30 (bs, 1H).

(Example 24) (R)-2-Bromo-3-phenylpropionic acid

Lithium bromide (0.13 g) was added to a mixture of 0.50 g of (S)-2-(4-chlorobenzenesulfonyloxy)-3-phenylpropionic acid obtained in Example 15 and 10 ml of toluene added thereto, the whole mixture was stirred at 60°C for 20 hours and then cooled to 20°C, 5 ml of pure water was then added and, after 30 minutes of stirring at 20°C, the organic layer was separated. The organic layer obtained contained 0.27 g of (R)-2-bromo-3-phenylpropionic acid (yield 80%, optical purity 96.0% ee). The rate of configuration inversion from methyl (S)-2-hydroxy-3-phenylpropionate was 96.0%.

(Example 25) (R)-2-Bromo-3-phenylpropionic acid

While adding lithium chloride to 67.25 g of an aqueous solution (pH 10.5) prepared from 11.50 g of (R)-2-bromo-3-phenylpropionic acid and 1.20 g of lithium hydroxide (1.0 mole per mole of (R)-2-bromo-3-phenylpropionic acid), the solution was sampled. The crystalline precipitate was filtered off, and each sample obtained was analyzed for the weight percent concentration (solubility) of the lithium (R)-2-bromo-3-phenylpropionate. The dependency of the solubility of lithium (R)-2-bromo-3-phenylpropionate on the lithium chloride concentration is shown in Fig. 2.

(Example 26) (R)-2-Bromo-3-phenylpropionic acid

Toluene (10 ml) and 2 ml of pure water were added to 5.72 g of (R)-2-bromo-3-phenylpropionic acid (obtained in Reference Example 2 (containing 5.00 g of (R)-2-bromo-3-phenylpropionic acid, 94.0% ee, and 0.17 g of cinnamic acid), the pH was then adjusted to 5.0 by adding 4.78 g of a 10% aqueous lithium hydroxide solution at an inside temperature of 10°C to crystallize and, then, the temperature was raised to 20°C. Following addition of 3 g of a 33% aqueous lithium chloride solution, the mixture was further stirred for 2 hours. The crystals formed were collected by filtration under reduced pressure and then washed with two 3-ml portions of toluene. Pure water (25 ml) was added to the wet crystals obtained for dissolution thereof, and the mixture was washed with two 25-ml portions of toluene. Toluene (25 ml) was added to this aqueous layer, the mixture was adjusted to pH 1 by adding 1.96 g of concentrated hydrochloric acid at an inside temperature of 10°C and, after 30 minutes of stirring at 10°C, the organic layer was separated. This organic layer was washed with three 10-ml portions of pure water and concentrated under reduced pressure. The thus-obtained concentrate (5.13 g) contained 4.77 g of (R)-2-bromo-3-phenylpropionic acid (yield 95%, optical purity 99.6% ee, cinnamic acid content not more than 0.01 g).

(Example 27) (R)-2-Bromo-3-phenylpropionic acid

Pure water (80 g) was added to 492 g of the concentrate obtained in Reference Example 3 (containing 200 g of (R) -2-bromo-3-phenylpropionic acid and 1.10 g of cinnamic acid) , and the pH was then adjusted to 4.8 by adding 187.8 g of a 10% aqueous lithium hydroxide solution over 4 hours at an inside temperature of 40°C to crystallize. In succession, 100 g of a 33% aqueous lithium chloride solution was added over 1 hour at an inside temperature of 40°C, and the mixture was then cooled to 20°C and stirred for 2 hours. The resulting crystals were collected by filtration under reduced pressure and then washed with two 200-ml portions of toluene. Pure water (1600 ml) was added to the wet crystals obtained for dissolution thereof, and the solution was washed with 500 ml of toluene. To this aqueous layer was added 1000 ml of toluene, the pH was adjusted to 1.5 by adding 77.4 g of concentrated hydrochloric acid at an inside temperature of 10°C and, after 30 minutes of stirring at 10°C, the organic layer was separated. This organic layer was washed with three 200-ml portions of pure water and then concentrated under reduced pressure. The thus-obtained concentrate (192.3 g) contained 175.9 g of (R)-2-bromo-3-phenylpropionic acid (yield 88%, optical purity 99.4% ee, cinnamic acid content not more than 0.1 g).

(Example 28) (R)-2-Bromopropionic acid

Lithium bromide (0.26 g) was added to a mixture prepared by adding 10 ml of toluene to 0.50 g of (S)-2-methanesulfonyloxypropionic acid (obtained in Example 16), the resulting mixture was stirred at 60°C for 1 hour and then cooled to 20°C, 5 ml of pure water was added and, after 30 minutes of stirring at 20°C, the organic layer was separated. The thus-obtained organic layer contained 0.19 g of (R) -2-bromopropionic acid (yield 45%, optical purity 92.3% ee) . The rate of configuration inversion from methyl L-lactate was 92.3%.

(Example 29) (R)-2-Bromo-3-phenylpropionic acid

Cyclohexylamine (0.43 g) was added to 18.3 g of an (R)-2-bromo-3-phenylpropionic acid/ethyl acetate solution separately obtained (containing 0.97 g of (R)-2-bromo-3-phenylpropionic acid, optical purity 94.0% ee) over 1 hour at an inside temperature of 40°C to crystallize, followedby further 2 hours of stirring. The resulting crystals were collected by filtration under reduced pressure and then washed with 3 ml of ethyl acetate. The thus-obtained wet crystals were dried under reduced pressure to give 1.28 g of dry crystals of (R)-2-bromo-3-phenylpropionic acid cyclohexylamine salt.
$^1$H-NMR ($CDCl_3$) δ (ppm): 1.10-1.37 (m, 5H), 1.62 (d, J = 11.7 Hz, 1H), 1.75 (d, J = 9.8 Hz, 2H), 1.95 (bs, 2H), 2.82 (t, J = 11.2 Hz, 1H), 3.16 (m, 1H), 3.46 (m, 1H), 4.35 (t, J = 7.3 Hz, 1H), 7.20-7.31 (m, 5H).
IR (KBr): 3403, 2942, 1632, 1561, 1453, 1437, 1389, 1308, 1281, 1171, 1082, 1042, 864, 750, 704, 669, 561, 519, 451, 407 ($cm^{-1}$).

The dry crystals obtained were added to a mixture of 5 ml of water and 5 ml of toluene, the pH was adjusted to 2.0 by addition of concentrated hydrochloric acid under ice cooling, and the organic layer was separated. The organic layer obtained contained 0.88 g of (R)-2-bromo-3-phenylpropionic acid (optical purity 96.5% ee).

(Example 30) (R)-2-Bromo-3-phenylpropionic acid

Dicyclohexylamine (0.79 g) was added to 10.2 g of an (R)-2-bromo-3-phenylpropionic acid/ethyl acetate solution separately obtained (containing 0.97 g of (R)-2-bromo-3-phenylpropionic acid, optical purity 94.0% ee) over 1 hour at an inside temperature of 40°C to crystallize, followed by further 2 hours of stirring. The resulting crystals were collected by filtration under reduced pressure and then washed with 3 ml of ethyl acetate. The thus-obtained wet crystals were dried under reduced pressure to give 1.05 g of dry crystals of (R)-2-bromo-3-phenylpropionic acid dicyclohexylamine salt.
$^1$H-NMR ($CDCl_3$) δ (ppm): 1.11-1.26 (m, 6H), 1.46 (d, J = 11.7 Hz, 4H), 1.64 (bs, 2H), 1.78 (d, J = 9.3 Hz, 4H), 1.98 (bs, 4H), 2.97 (t, J = 7.8 Hz, 2H), 3.19 (m, 1H), 3.50 (m, 1H), 4.37 (t, J = 7.6 Hz, 1H), 7.19-7.23 (m, 1H), 7.25-7.28 (m, 4H).
IR (KBr) : 3569, 2942, 2855, 1632, 1543, 1378, 1256, 1152, 1082, 1063, 1049, 1034, 918, 897, 885, 851, 791, 741, 727, 706, 695, 633, 594, 556, 515, 488, 448, 421 ($cm^{-1}$).

The dry crystals obtained were added to 5 ml of ethyl acetate, the solution was washed, under ice cooling, with two 5-ml portions of a 5% aqueous potassium hydrogensulfate solution and then with 5 ml of water to give an organic layer. The thus-obtained organic layer contained 0.58 g of (R)-2-bromo-3-phenylpropionic acid (optical purity 96.9% ee).

(Example 31) (R)-2-Bromo-3-phenylpropionic acid

Toluene (5 ml) was added to 1.14 g of (R)-2-bromo-3-phenylpropionic acid separately obtained (containing 1.0 g of (R)-2-bromo-3-phenylpropionic acid, optical purity 94.0% ee), the inside temperature was raised to 50°C, and a mixed solution composed of 0.53 g of (S)-1-phenylethylamine and 5 ml of toluene was added slowly. After 30 minutes of stirring, the mixture was cooled to 5°C and, after precipitation of crystals, the mixture was further stirred at 5°C for 1 hour. The crystals formed were collected by filtration under reduced pressure and then washed with 5 ml of cold toluene. The thus-obtained wet crystals were dried under reduced pressure to give 1.13 g of dry crystals of (R)-2-bromo-3-phenylpropionic acid (S)-1-phenylethylamine salt.
$^1$H-NMR ($CDCl_3$) δ (ppm): 1.47 (d, J = 6.8 Hz, 3H), 2.92 (m, 1H) , 3.18 (m, 1H), 4.04 (t, J = 7.3 Hz, 1H), 4.17 (q, J = 6.8 Hz, 1H), 5.73 (bs, 1H), 7.09-7.12 (m, 2H), 7.18-7.31 (m, 6H), 7.37-7.40 (m, 2H).
IR (KBr) : 3411, 2925, 1626, 1561, 1497, 1451, 1383, 1252, 1202, 1138, 1094, 1075, 1038, 916, 837, 768, 754, 700, 683, 563, 538, 494, 480, 403 ($cm^{-1}$).

Toluene (5 ml) and 5 ml of water were added to the dry crystals obtained, the pH was adjusted to 2.0 by addition of concentrated hydrochloric acid under ice cooling, and the organic layer was separated. The organic layer

obtained contained 0.73 g of (R)-2-bromo-3-phenylpropionic acid (optical purity 99.1% ee).

(Example 32) (R)-2-Bromo-3-phenylpropionic acid

Toluene (10 ml) was added to 1.71 g of oily (R)-2-bromo-3-phenylpropionic acid separately obtained (containing 1.5 g of (R) -2-bromo-3-phenylpropionic acid, 97.0% ee), and a mixed solution composed of 1.17 g of L-phenylalanine methyl ester and 10 ml of toluene was added slowly to crystallize. After 1 hour of stirring, the crystals formed were collected by filtration under reduced pressure and then washed with 5 ml of cold toluene. The thus-obtained wet crystals were dried under reduced pressure to give 2.43 g of dry crystals of (R)-2-bromo-3-phenylpropionic acid L-phenylalanine methyl ester salt.
$^1$H-NMR ($CDCl_3$) $\delta$ (ppm): 3.03 (m, 1H), 3.13 (m, 1H), 3.17 (m, 1H), 3.43 (m, 1H), 3.73 (s, 3H), 3.95 (t, J = 6.1 Hz, 1H), 4.38 (t, J = 7.3 Hz, 1H), 7.14-7.18 (m, 2H), 7.22-7.32 (m, 8H).
IR (KBr): 3569, 2951, 2168, 1749, 1619, 1570, 1509, 1455, 1439, 1397, 1379, 1343, 1252, 1206, 1171, 1129, 1078, 1053, 1030, 945, 912, 858, 770, 743, 708, 700, 633, 600, 559, 500, 459, 421 ($cm^{-1}$).
Toluene (5 ml) and 5 ml of water were added to the dry crystals obtained, the pH was adjusted to 2.0 by addition of concentrated hydrochloric acid under ice cooling, and the organic layer was separated. The organic layer obtained contained 1.34 g of (R)-2-bromo-3-phenylpropionic acid (optical purity 98.5% ee).

(Example 33) (R)-2-Bromo-3-phenylpropionic acid

Toluene (10 ml) was added to 1.71 g of oily (R)-2-bromo-3-phenylpropionic acid separately obtained (containing 1.5 g of (R) -2-bromo-3-phenylpropionic acid, 97.0% ee), and a mixed solution composed of 1.27 g of L-phenylalanine ethyl ester and 10 ml of toluene was added slowly to crystallize. After 1 hour of stirring, the crystals formed were collected by filtration under reduced pressure and then washed with 5 ml of cold toluene. The thus-obtained wet crystals were dried under reduced pressure to give 2.50 g of dry crystals of (R)-2-bromo-3-phenylpropionic acid L-phenylalanine ethyl ester salt.
$^1$H-NMR ($CDCl_3$) $\delta$ (ppm): 1.22 (t, J = 7.1 Hz, 3H), 3.08-3.12 (m, 2H), 3.17 (m, 1H), 3.43 (m, 1H), 3.97 (t, J= 6.3 Hz, 1H), 4.16 (q, J = 7.3 Hz, 2H), 4.38 (t, J = 7.3 Hz, 1H), 7.17-7.21 (m, 2H), 7.22-7.32 (m, 8H).
IR (KBr): 3457, 2979, 1740, 1665, 1601, 1536, 1497, 1458, 1383, 1331, 1314, 1266, 1229, 1181, 1167, 1144, 1098, 1076, 1059, 1030, 1019, 990, 955, 918, 853, 785, 747, 714, 700, 629, 598, 561, 504, 401 ($cm^{-1}$).
Toluene (5 ml) and 5 ml of water were added to the dry crystals obtained, the pH was adjusted to 2.0 by addition of concentrated hydrochloric acid under ice cooling, and the organic layer was separated. The organic layer obtained contained 1.34 g of (R)-2-bromo-3-phenylpropionic acid (optical purity 98.5% ee).

(Example 34) (R)-2-Bromo-3-phenylpropionic acid

Toluene (10 ml) was added to 1.43 g of D-phenylalaninamide, and 2.3 g of concentrated (R)-2-bromo-3-phenylpropionic acid (optical purity 94.0%) dissolved in 10 ml of toluene was then added at room temperature. After 1 hour of stirring, the resulting crystals were collected by filtration under reduced pressure and then washed with 5 ml of toluene. The thus-obtained wet crystals were dried under reduced pressure to give 1.72 g of dry crystals of (R) -2-bromo-3-phenylpropionic acid D-phenylalaninamide salt.
$^1$H-NMR ($CDCl_3$) $\delta$ (ppm): 2.85 (m, 1H), 3.05-3.14 (m, 2H), 3.36 (m, 1H) , 3.82(t, J= 6. 8 Hz, 1H), 4.33 (t, J=7.6Hz, 1H), 6.17 (bs, 1H), 6.98 (bs, 2H), 7.12-7.25 (m, 10H).
IR (KBr) : 3420, 3031, 1696, 1624, 1584, 1495, 1456, 1435, 1399, 1347, 1323, 1306, 1266, 1204, 1123, 1078, 1046, 936, 916, 826, 806, 745, 700, 644, 600, 575, 519, 473, 451, 419 ($cm^{-1}$).
Toluene (5 ml) and 5 ml of water were added to the dry crystals obtained, the pH was adjusted to 2.0 by addition of concentrated hydrochloric acid under ice cooling, and the organic layer was separated. The organic layer obtained contained 0.98 g of (R)-2-bromo-3-phenylpropionic acid (optical purity 96.2% ee).

INDUSTRIAL APPLICABILITY

The invention, which has the constitution described hereinabove, makes it possible to produce an optically active 2-bromocarboxylic acid and an optically active 2-sulfonyloxycarboxylic acid, which are high in optical purity and in chemical purity and are important in the production of medicinal chemicals and so forth, in an economical and efficient manner.

## Claims

**1.** A process for producing an optically active 2-bromocarboxylic acid represented by the general formula (2) :

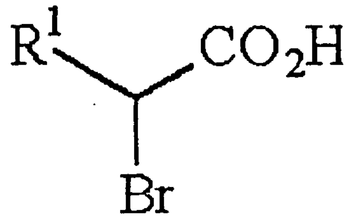

(2)

in the formula, $R^1$ represents an alkyl group containing 1 to 12 carbon atoms, which may optionally be substituted, an aryl group containing 6 to 14 carbon atoms, which may optionally be substituted, or an aralkyl group containing 7 to 15 carbon atoms, which may optionally be substituted,

which process comprises reacting an optically active 2-sulfonyloxycarboxylic acid represented by the general formula (1):

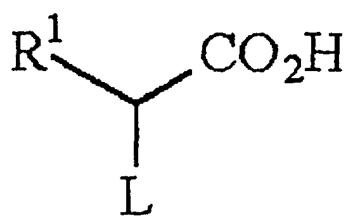

(1)

in the formula, $R^1$ is as defined above and L represents a sulfonyloxy group, with a metal bromide for bromination with configuration inversion at position 2.

**2.** The process according to Claim 1,

wherein the metal bromide is lithium bromide.

**3.** The process according to Claim 1 or 2,

wherein a hydrocarbon or an ether is used as the reaction solvent.

**4.** The process according to any one of Claims 1 to 3,

wherein the optically active 2-bromocarboxylic acid formed is extracted with toluene.

**5.** The process according to any one of Claims 1 to 4,

wherein the rate of configuration inversion is not less than 90%.

**6.** The process according to any one of Claims 1 to 5,

wherein the optically active 2-sulfonyloxycarboxylic acid (1) is one obtained by deprotecting an optically active 2-sulfonyloxycarboxylic acid ester represented by the general formula (3):

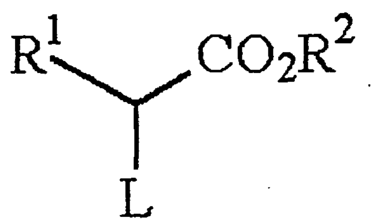

(3)

in the formula, $R^1$ and L are as defined above, and $R^2$ represents a univalent organic group included in the structure

represented by -$COOR^2$ and capable of serving as an ester type protective group for the carboxyl group.

**7.** The process according to Claim 6,
wherein the optically active 2-sulfonyloxycarboxylic acid ester (3) is one obtained by converting an optically active 2-hydoxycarboxylic acid represented by the general formula (4) :

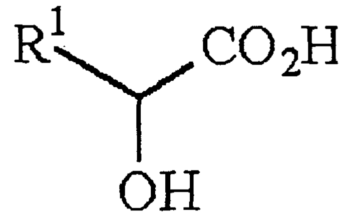

(4)

in the formula, $R^1$ is as defined above, to the corresponding optically active 2-hydroxycarboxylic acid ester represented by the general formula (5):

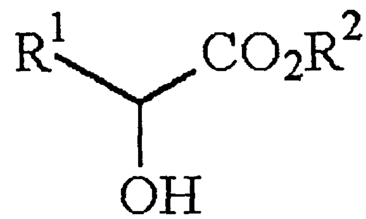

(5)

in the formula, $R^1$ and $R^2$ are as defined above, by esterification and treating said optically active 2-hydroxycarboxylic acid ester with a leaving group introducing agent.

**8.** The process according to Claim 6 or 7,
wherein the deprotection is carried out under acidic conditions.

**9.** The process according to Claim 8,
wherein the deprotection under acidic conditions is carried out by a method comprising reacting with a carboxylic acid in the presence of a strong acid, or by a method comprising carrying out the reaction in an aqueous medium containing an organic solvent highly compatible with water in the presence of a strong acid.

**10.** The process according to any one of Claims 1 to 9,
wherein $R^1$ is a benzyl group.

**11.** A process for producing an optically active 2-bromocarboxylic acid,
which comprises extracting and/or washing an optically active 2-bromocarboxylic acid represented by the general formula (2):

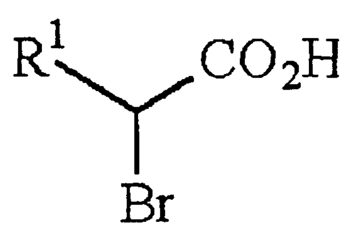

(2)

in the formula, $R^1$ represents an alkyl group containing 1 to 12 carbon atoms, which may optionally be substituted,

an aryl group containing 6 to 14 carbon atoms, which may optionally be substituted, or an aralkyl group containing 7 to 15 carbon atoms, which may optionally be substituted, with a mixed solvent system composed of an aromatic hydrocarbon and water to thereby remove the corresponding coexisting optically active 2-sulfonyloxycarboxylic acid (1) and/or optically active 2-hydroxycarboxylic acid (4).

**12.** The process according to Clam 11,
wherein the aromatic hydrocarbon is an aromatic hydrocarbon containing 6 to 12 carbon atoms.

**13.** The process according to Claim 12,
wherein the aromatic hydrocarbon is toluene.

**14.** The process according to any one of Claims 11 to 13,
wherein $R^1$ is a benzyl group.

**15.** A process for isolating/purifying an optically active 2-bromocarboxylic acid,
which comprises, in isolating and purifying an optically active 2-bromocarboxylic acid represented by the general formula (2):

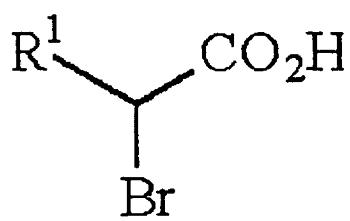

(2)

in the formula, $R^1$ represents an alkyl group containing 1 to 12 carbon atoms, which may optionally be substituted, an aryl group containing 6 to 14 carbon atoms, which may optionally be substituted, or an aralkyl group containing 7 to 15 carbon atoms, which may optionally be substituted, and containing at least the optical isomer thereof as an impurity, the step of crystallizing and separating said optically active 2-bromocarboxylic acid in the form of a salt with a base.

**16.** The process according to Claim 15,
wherein the salt of the optically active 2-bromocarboxylic acid with a base is a metal salt or an ammonium salt of the optically active 2-bromocarboxylic acid.

**17.** The process according to Claim 16,
wherein the salt of the optically active 2-bromocarboxylic acid with a base is an alkali metal salt of the optically active 2-bromocarboxylic acid.

**18.** The process according to Claim 17,
wherein the salt of the optically active 2-bromocarboxylic acid with a base is the lithium salt of the optically active 2-bromocarboxylic acid.

**19.** The process according to any one of Claims 16 to 18,
wherein the base is used in an amount of 0.8 to 1.2 equivalents relative to the optically active 2-bromocarboxylic acid.

**20.** The process according to any one of Claims 16 to 19,
wherein the crystallization is carried out in the presence of water.

**21.** The process according to Claim 20,
wherein the crystallization is carried out under weakly acidic to basic conditions.

**22.** The process according to Claim 21,

wherein the crystallization is carried out at a pH of 4 to 7.

**23.** The process according to any one of Claims 20 to 22,
wherein the crystallization is carried out at a temperature of not lower than 30°C.

**24.** The process according to any one of Claims 20 to 23,
wherein, in carrying out the crystallization, a salt for salting out is caused to coexist and/or an organic solvent is used in combination.

**25.** The process according to Claim 24,
wherein the cation of the salt for salting out is the same as the cation in the salt of the optically active 2-bromocarboxylic acid with a base to be salted out.

**26.** The process according to Claim 24 or 25,
wherein the salt for salting out is an alkali metal salt.

**27.** The process according to any one of Claims 24 to 26,
wherein the salt of the optically active 2-bromocarboxylic acid with a base is the lithium salt of the optically active 2-bromocarboxylic acid and the salt for salting out is lithium chloride or lithium sulfate.

**28.** The process according to any one of Claims 24 to 27,
wherein the salt for salting out is used at a concentration of not lower than 5% by weight relative to water.

**29.** The process according to Claim 24,
wherein the organic solvent used in combination is an organic solvent low in compatibility with water.

**30.** The process according to Claim 29,
wherein the organic solvent low in compatibility with water is an aromatic hydrocarbon.

**31.** The process according to Claim 30,
wherein the aromatic hydrocarbon is an aromatic hydrocarbon containing 6 to 12 carbon atoms.

**32.** The process according to Claim 31,
wherein the aromatic hydrocarbon containing 6 to 12 carbon atoms is toluene.

**33.** The process according to any one of Claims 24 to 32,
wherein, in the step crystallization, the organic solvent low in compatibility with water is used in an amount not exceeding 4 parts by weight per part by weight of the optically active 2-bromocarboxylic acid.

**34.** The process according to any one of Claims 24 to 33,
wherein, in the step of crystallization, water is used in an amount not exceeding 20 parts by weight per part by weight of the optically active 2-bromocarboxylic acid.

**35.** The process according to Claim 24,
wherein the organic solvent used in combination is an organic solvent highly compatible with water.

**36.** The process according to Claim 35,
wherein the solvent highly compatible with water is a ketone.

**37.** The process according to Claim 36,
wherein the ketone is acetone.

**38.** The process according to Claim 15,
wherein the salt of the optically active 2-bromocarboxylic acid with a base is an amine salt or the ammonium salt of the optically active 2-bromocarboxylic acid.

**39.** The process according to Claim 38,
wherein the amine salt of the optically active 2-bromocarboxylic acid is an alkylamine salt of the optically

active 2-bromocarboxylic acid.

**40.** The process according to Claim 39,
wherein the alkylamine salt of the optically active 2-bromocarboxylic acid is the cyclohexylamine salt or dicyclohexylamine salt of the optically active 2-bromocarboxylic acid.

**41.** The process according to Claim 38,
wherein the amine salt of the optically active 2-bromocarboxylic acid is a salt of the optically active 2-bromocarboxylic acid with an amine containing a phenyl group.

**42.** The process according to Claim 41,
wherein the salt of the optically active 2-bromocarboxylic acid with an amine containing a phenyl group is a salt of the optically active 2-bromocarboxylic acid with an aralkylamine, an amino acid ester containing a phenyl group, or an amino acid amide containing a phenyl group.

**43.** The process according to Claim 42,
wherein the salt of the optically active 2-bromocarboxylic acid with an aralkylamine is the 1-phenylethylamine salt of the optically active 2-bromocarboxylic acid.

**44.** The process according to Claim 42,
wherein the salt of the optically active 2-bromocarboxylic acid with an amino acid ester containing a phenyl group is a phenylalanine ester salt of the optically active 2-bromocarboxylic acid or a phenylglycine ester salt of the optically active 2-bromocarboxylic acid.

**45.** The process according to Claim 42,
wherein the salt of the optically active 2-bromocarboxylic acid with an amino acid amide containing a phenyl group is the phenylalaninamide salt of the optically active 2-bromocarboxylic acid or the phenylglycinamide salt of the optically active 2-bromocarboxylic acid.

**46.** The process according to any one of Claims 38 to 45,
wherein the crystallization is carried out in the presence of an organic solvent.

**47.** The process according to Claim 46,
wherein the organic solvent is acetone, methylene chloride, ethyl acetate or toluene.

**48.** The process according to any one of Claims 15 to 47,
which comprises the step of further converting the salt of the isolated/purified optically active 2-bromocarboxylic acid with a base to the corresponding free optically active 2-bromocarboxylic acid.

**49.** The process according to Claim 48,
wherein the step of converting to the free optically active 2-bromocarboxylic acid comprises converting the salt of the isolated/purified 2-bromocarboxylic acid with a base to the free optically active 2-bromocarboxylic acid by neutralization using an acid and then recovering the free optically active 2-bromocarboxylic acid or a solution thereof by extraction with an organic solvent, if necessary followed by removal of the solvent.

**50.** The process according to Claim 49,
wherein the organic solvent is toluene.

**51.** The process according to any one of Claims 15 to 50,
wherein $R^1$ in the general formula (2) is a benzyl group.

**52.** The process according to Claim 15,
wherein the optically active 2-bromocarboxylic acid is one obtained by the process according to any one of Claims 1 to 14.

**53.** A free optically active 2-bromocarboxylic acid or a salt thereof as obtained by the process of any one of Claims 15 to 52 and having an optical purity of not lower than 97% ee.

**54.** A crystalline salt of an optically active 2-bromo-3-phenylpropionic acid with a base.

**55.** The crystalline salt according to Claim 54,
wherein the salt of the optically active 2-bromo-3-phenylpropionic acid with a base is a metal salt or the ammonium salt of the optically active 2-bromo-3-phenylpropionic acid.

**56.** The crystalline salt according to Claim 55,
wherein the salt of the optically active 2-bromo-3-phenylpropionic acid with a base is an alkali metal salt of the optically active 2-bromo-3-phenylpropionic acid.

**57.** The crystalline salt according to Claim 56,
wherein the salt of the optically active 2-bromo-3-phenylpropionic acid with a base is the lithium salt of the optically active 2-bromo-3-phenylpropionic acid.

**58.** The crystalline salt according to Claim 54,
wherein the salt of the optically active 2-bromo-3-phenylpropionic acid with a base is an amine salt of the optically active 2-bromo-3-phenylpropionic acid.

**59.** The crystalline salt according to Claim 58,
wherein the salt of the optically active 2-bromo-3-phenylpropionic acid with a base is an alkylamine salt of the optically active 2-bromo-3-phenylpropionic acid.

**60.** The crystalline salt according to Claim 59,
wherein the alkylamine salt of the optically active 2-bromo-3-phenylpropionic acid is the cyclohexylamine salt or dicyclohexylamine salt of the optically active 2-bromo-3-phenylpropionic acid.

**61.** The crystalline salt according to Claim 58,
wherein the salt of the optically active 2-bromo-3-phenylpropionic acid with a base is a salt of the optically active 2-bromo-3-phenylpropionic acid with an amine containing a phenyl group.

**62.** The crystalline salt according to Claim 61,
wherein the salt of the optically active 2-bromo-3-phenylpropionic acid with an amine containing a phenyl group is a salt of the optically active 2-bromo-3-phenylpropionic acid with an aralkylamine, an amino acid ester containing a phenyl group, or an amino acid amide containing a phenyl group.

**63.** The crystalline salt according to Claim 62,
wherein the salt of the optically active 2-bromo-3-phenylpropionic acid with an aralkylamine is the 1-phenylethylamine salt of the optically active 2-bromo-3-phenylpropionic acid.

**64.** The crystalline salt according to Claim 62,
wherein the salt of the optically active 2-bromo-3-phenylpropionic acid with an amino acid ester containing a phenyl group is a phenylalanine ester salt of the optically active 2-bromo-3-phenylpropionic acid or a phenylglycine ester salt of the optically active 2-bromo-3-phenylpropionic acid.

**65.** The crystalline salt according to Claim 62,
wherein the salt of the optically active 2-bromo-3-phenylpropionic acid with an amino acid amide containing a phenyl group is the phenylalaninamide salt of the optically active 2-bromo-3-phenylpropionic acid or the phenylglycinamide salt of the optically active 2-bromo-3-phenylpropionic acid.

**66.** A process for producing an optically active 2-sulfonyloxycarboxylic acid represented by the general formula (1):

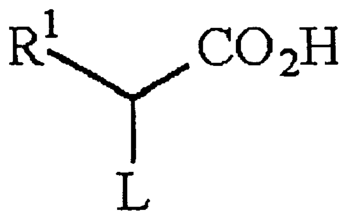

(1)

in the formula, $R^1$ represents an alkyl group containing 1 to 12 carbon atoms, which may optionally be substituted, an aryl group containing 6 to 14 carbon atoms, which may optionally be substituted, or an aralkyl group containing 7 to 15 carbon atoms, which may optionally be substituted, and L represents a sulfonyloxy group,

which comprises deprotecting an optically active 2-sulfonyloxycarboxylic acid ester represented by the general formula (3):

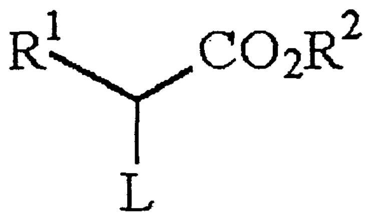

(3)

in the formula, $R^1$ and L are as defined above and $R^2$ represents a univalent organic group included in the structure represented by $-COOR^2$ and capable of serving as an ester type protective group for the carboxyl group, under acidic conditions.

**67.** The process according to Claim 66,
wherein the deprotection under acidic conditions is carried out by a method comprising reacting with a carboxylic acid in the presence of a strong acid, or by a method comprising carrying out the reaction in an aqueous medium containing an organic solvent highly compatible with water in the presence of a strong acid.

**68.** The process according to Claim 66 or 67,
wherein the optically active 2-sulfonyloxycarboxylic acid formed is neutralized with sodium hydroxide in the presence of water and said carboxylic acid is recovered in the form of the sodium salt.

**69.** The process according to any one of Claims 66 to 68,
wherein the optically active 2-sulfonyloxycarboxylic acid formed is extracted with tert-butyl methyl ether.

**70.** The process according to any one of Claims 66 to 69,
wherein the optically active 2-sulfonyloxycarboxylic acid formed is crystallized using toluene.

**71.** The process according to any one of Claims 66 to 70,
wherein the rate of configuration retention in the step of deprotection is not less than 90%.

**72.** The process according to Claim 71,
wherein the rate of configuration retention in the step of deprotection is not less than 97%.

**73.** The process according to any one of Claims 66 to 72,
wherein the optically active 2-sulfonyloxycarboxylic acid ester is one obtained by converting an optically active 2-hydoxycarboxylic acid represented by the general formula (4) :

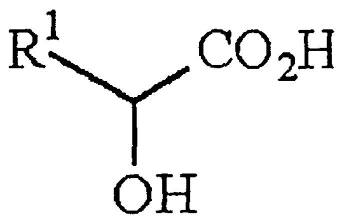

(4)

in the formula, $R^1$ is as defined above, to the corresponding optically active 2-hydroxycarboxylic acid ester represented by the general formula (5):

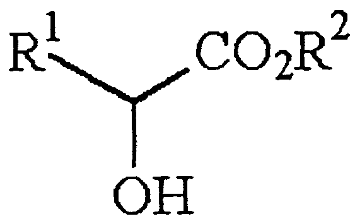

(5)

in the formula, $R^1$ and $R^2$ are as defined above, by esterification and treating said optically active 2-hydroxycarboxylic acid ester with a leaving group introducing agent.

**74.** The process according to any one of Claims 66 to 73,
wherein $R^1$ is a benzyl group.

**75.** A process for producing an optically active 2-hydroxycarboxylic acid ester,
which comprises extracting and/or washing an optically active 2-hydroxycarboxylic acid ester represented by the general formula (5):

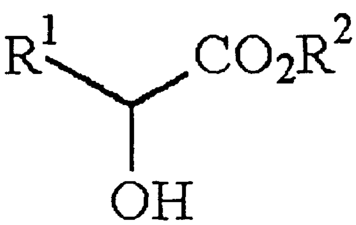

(5)

in the formula, $R^1$ represents an alkyl group containing 1 to 12 carbon atoms, which may optionally be substituted, an aryl group containing 6 to 14 carbon atoms, which may optionally be substituted, or an aralkyl group containing 7 to 15 carbon atoms, which may optionally be substituted, and $R^2$ represents a univalent organic group included in the structure represented by $-COOR^2$ and capable of serving as an ester type protective group for the carboxyl group, with a mixed solvent system composed of an organic solvent and water under weakly acidic to basic conditions to thereby eliminate the coexisting optically active 2-hydroxycarboxylic acid.

**76.** The process according to Claim 75,
wherein the organic solvent is a hydrocarbon solvent or an ester solvent.

**77.** The process according to Claim 76,
wherein the organic solvent is a hydrocarbon solvent.

**78.** The process according to Claim 77,
wherein the hydrocarbon solvent is an aromatic hydrocarbon solvent or an aliphatic hydrocarbon solvent.

**79.** The process according to any one of Claims 75 to 78,
wherein the pH in the step of extraction and/or washing is not lower than 4.

**80.** The process according to any one of Claims 75 to 79,
wherein the optically active 2-hydroxycarboxylic acid ester represented by the general formula (5) is one produced by reacting an optically active 2-hydroxycarboxylic acid represented by the general formula (4):

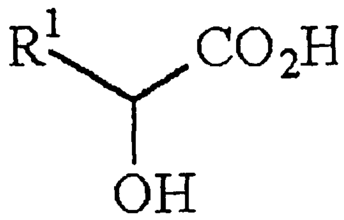

(4)

in the formula, $R^1$ is as defined above, with an alcohol and an acid or thionyl chloride.

**81.** A process for producing an optically active 2-hydroxycarboxylic acid ester,
which comprises crystallizing an optically active 2-hydroxycarboxylic acid ester represented by the general formula (5):

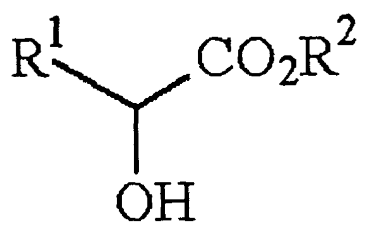

(5)

in the formula, $R^1$ represents an alkyl group containing 1 to 12 carbon atoms, which may optionally be substituted, an aryl group containing 6 to 14 carbon atoms, which may optionally be substituted, or an aralkyl group containing 7 to 15 carbon atoms, and which may optionally be substituted, and $R^2$ represents a univalent organic group included in the structure represented by $-COOR^2$ and capable of serving as an ester type protective group for the carboxyl group, and containing at least the optical isomer thereof as an impurity, using an aliphatic hydrocarbon solvent to recover said ester as crystals improved in optical purity.

**82.** The process according to Claim 81,
wherein an aromatic hydrocarbon solvent and/or an ester solvent is used as an auxiliary solvent.

**83.** The process according to Claim 81 or 82,
wherein the aliphatic hydrocarbon solvent is added to the optically active 2-hydroxycarboxylic acid ester gradually.

**84.** The process according to any one of Claims 81 to 83,
wherein, at the time of completion of the crystallization, the volume of the aliphatic hydrocarbon solvent amounts to at least one half the whole solvent volume.

**85.** The process according to any one of Claims 81 to 84,
wherein the crystallization operation is carried out at a temperature not higher than 60°C.

**86.** The process according to any one of Claims 81 to 85,
wherein the optically active 2-hydroxycarboxylic acid ester to be crystallized is one produced by the process according to Claim 75.

**87.** The process according to any one of Claims 75 to 86, wherein $R^1$ in the general formula (5) is a benzyl group.

Fig.1

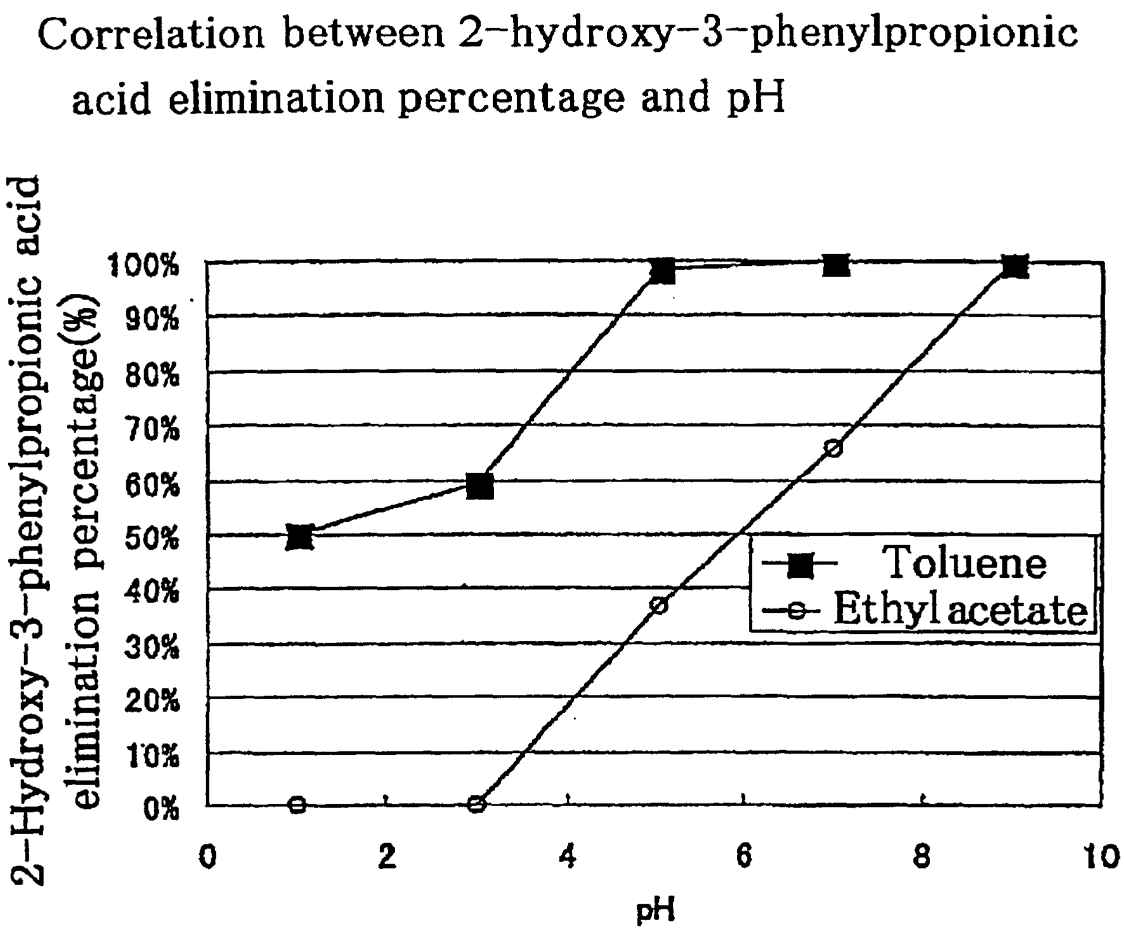
Correlation between 2-hydroxy-3-phenylpropionic acid elimination percentage and pH
2-Hydroxy-3-phenylpropionic acid elimination percentage(%)
100%
90%
80%
70%
60%
50%
40%
30%
20%
10%
0%
0
2
4
6
8
10
pH
Toluene
Ethyl acetate

Fig.2

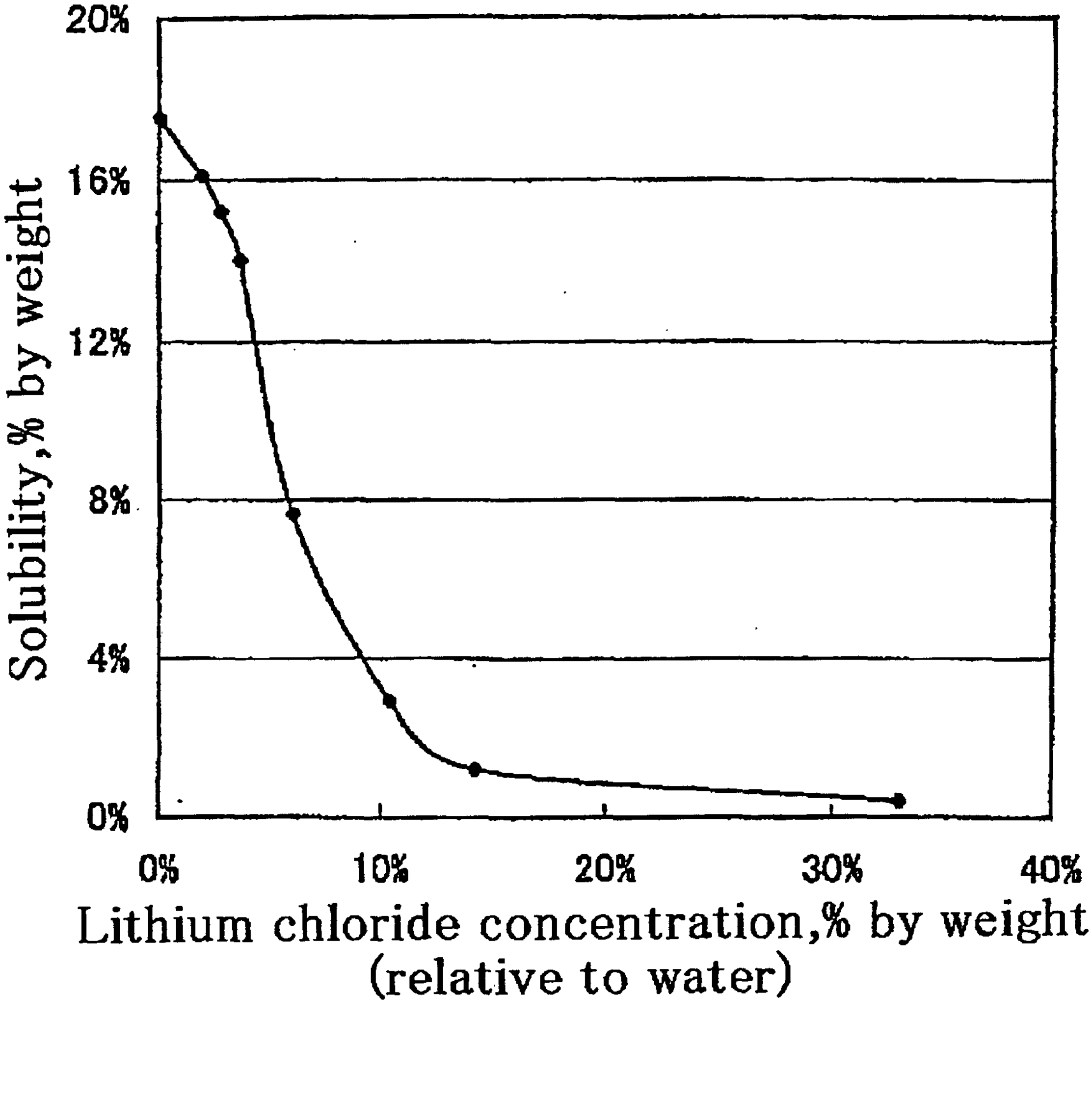
20%
16%
12%
8%
4%
0%
Solubility,% by weight
0%
10%
20%
30%
40%
Lithium chloride concentration,% by weight
(relative to water)

## INTERNATIONAL SEARCH REPORT

International application No.
PCT/JP02/08098

A. CLASSIFICATION OF SUBJECT MATTER
Int.Cl$^7$ C07C51/363, 57/58, 53/19, 67/08, 69/675, 69/732, C07B55/00, C07C309/70

According to International Patent Classification (IPC) or to both national classification and IPC

B. FIELDS SEARCHED

Minimum documentation searched (classification system followed by classification symbols)
Int.Cl$^7$ C07C51/363, 57/58, 53/19, 67/08, 69/675, 69/732, C07B55/00, C07C309/70

Documentation searched other than minimum documentation to the extent that such documents are included in the fields searched

Electronic data base consulted during the international search (name of data base and, where practicable, search terms used)
CASREACT(STN)

C. DOCUMENTS CONSIDERED TO BE RELEVANT

| Category* | Citation of document, with indication, where appropriate, of the relevant passages | Relevant to claim No. |
|---|---|---|
| Y | HANESSIAN S, "Design and Reactivity of Organic Functional Groups - 2-Pyridylsulfonates as Nucleofugal Esters", Heterocycles, 1989, Vol.28, No.2, pages 1115 to 1120 | 1-10 |
| Y | JOSEPH K. et al., "Dependence of rotatory power on chemical constitution. XXIV.", J.Chem.Soc., 1925, Vol.127, pages 399 to 417. (abstract) CASREACT [online]: Chemical Abstracts Service, Columbus, Ohio, USA. [retrieved on 25 November, 2002 (25.11.02)]. Retrieved from: The Scientific & Technical Information Network, Columbus, Ohio, USA. CASREACT Accession No.19:10433 | 1-10 |

[X] Further documents are listed in the continuation of Box C.   [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 04 December, 2002 (04.12.02) | 10 December, 2002 (10.12.02) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP02/08098

C (Continuation). DOCUMENTS CONSIDERED TO BE RELEVANT

| Category* | Citation of document, with indication, where appropriate, of the relevant passages | Relevant to claim No. |
|---|---|---|
| Y | WO 99/42431 A1 (ZAMBON GROUP S.P.A.), 26 August, 1999 (26.08.99), Claims & EP 1056707 A1 & US 6103931 A | 1-10 |
| Y<br>A | EP 67698 A2 (SAGAMI CHEMICAL RESEARCH CENTER), 22 December, 1982 (22.12.82), Page 13; examples 1, 2 & JP 58-10525 A & US 4539420 A | 6-10<br>1-5 |
| Y<br>A | EP 81993 A2 (SYNTEX PHARMACEUTICALS), 22 June, 1983 (22.06.83), Examples 1 to 3 & JP 58-135833 A & US 4605758 A & US 4749804 A & US 4912254 A & US 5210258 A | 6-10<br>1-5 |
| Y<br>A | JP 10-14590 A (Kaneka Corp.), 20 January, 1998 (20.01.98), Claims (Family: none) | 6-10<br>1-5 |
| Y<br>A | Edited by CSJ: The Chemical Society of Japan, 4th edition, "Jikken Kagaku Koza 22 Yuki Gosei IV -San·Amino-san·Peptide-, Maruzen Co., Ltd., 30 November, 1992 (30.11.92), page 7 | 6-10<br>1-5 |

Form PCT/ISA/210 (continuation of second sheet) (July 1998)

**INTERNATIONAL SEARCH REPORT**

International application No.
PCT/JP02/08098

Continuation of Box No. II of continuation of first sheet(1)

base; the compound (2) obtained by the process which is in a free form or a salt thereof; and crystals of a salt of the optically active 2-bromopropionic acid with a base, the salt being an intermediate in the isolation/purification method.
d. Claims 66 to 74: a process for producing the compound (1) from an optically active sulfonyloxycarboxylic ester (3) through elimination of the protective group.
e. Claims 75 to 80: a process for producing a 2-hydroxycarboxylic ester (5) by removing an optically active 2-hydroxycarboxylic acid through extraction and/or washing.
f. Claims 81 to 87: a process for producing through crystallization the compound (5) as crystals with a heightened optical purity.

Among these groups, there is no relationship involving any special technical feature common to these. Consequently, these groups are not considered to be so linked as to form a single general inventive concept.
Therefore, the number of inventions disclosed in the claims of this international application is 6.

Form PCT/ISA/210 (extra sheet) (July 1998)

## INTERNATIONAL SEARCH REPORT

International application No.
PCT/JP02/08098

**Box I Observations where certain claims were found unsearchable (Continuation of item 1 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

The subject matters of the claims are classified into the following groups.

a. Claims 1 to 10: a process for producing an optically active 2-bromocarboxylic acid (2) by the bromination of an optically active 2-sulfonyloxycarboxylic acid (1).

b. Claims 11 to 14: a process for producing the compound (2) by removing the compound (1) and/or an optically active 2-hydroxycarboxylic acid (4) through extraction and/or washing.

c. Claims 15 to 65: a method of isolating/purifying the compound (2) which comprises crystallizing and separating the compound (2) as a salt with a

(continued to extra sheet)

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.: 1 to 10

**Remark on Protest** ☐ The additional search fees were accompanied by the applicant's protest.
☐ No protest accompanied the payment of additional search fees.

Form PCT/ISA/210 (continuation of first sheet (1)) (July 1998)